# EUROPEAN PATENT APPLICATION

(11) **EP 2 131 193 A1**
(43) Date of publication of application: **09.12.2009**
(21) Application number: 08721401.1
(22) Date of filing: 05.03.2008
(51) Int. Cl.: G01N 33/15, G01N 33/50

(54) **METHOD FOR EXAMINATION OF ACTIVITY OF ANTI-CANCER AGENT EMPLOYING REDUCTION IN EXPRESSION LEVEL OF GENE AS MEASURE**

(30) Priority: 05.03.2007 US 904775 P; 01.10.2007 US 960486 P
(71) Applicant: Eisai R&D Management Co., Ltd., Tokyo 112-8088 (JP)
(72) Inventor: MIZUI, Yoshiharu, Tsukuba-shi Ibaraki 300-2635 (JP); HATA, Naoko, Tsukuba-shi Ibaraki 300-2635 (JP); IWATA, Masao, Tsukuba-shi Ibaraki 300-2635 (JP); SUGANO, Rie, Tsukuba-shi Ibaraki 300-2635 (JP); TORITSUKA, Naoki, Tsukuba-shi Ibaraki 300-2635 (JP); YOSHIDA, Taku, Tsukuba-shi Ibaraki 300-2635 (JP); UESUGI, Mai, Tsukuba-shi Ibaraki 300-2635 (JP); KADOWAKI, Tadashi, Tsukuba-shi Ibaraki 300-2635 (JP)
(74) Representative: Woods, Geoffrey Corlett
(86) International application number: PCT/JP2008/053981
(87) International publication number: WO 2008/111466

(57) **Abstract**

An object of the present invention is to provide a method, a probe, a primer, an antibody, a reagent, and a kit for assaying an action of a pladienolide derivative to a living subject. According to the present invention, there is provided a method for assaying an action of the pladienolide derivative using a decrease in gene expression level as an index.

## Description

### BACKGROUND OF THE INVENTION

### Field of the Invention

The present invention relates to a method for assaying an action of an antitumor agent using a decrease in gene expression level as an index, more particularly, a method for assaying an action of an antitumor agent using a decrease in the expression level of mRNA or the expression level of a protein as an index.

### Background Art

Pladienolide derivatives are derivatives of natural pladienolide. Since pladienolide exhibits an excellent antitumor activity (Mizui et al., 2004, J. Antibiotics 577: 188-196), search for a compound with higher antitumor activity has been performed to find the pladienolide derivatives (WO2002/060890 and W02003/099813).

### SUMMARY OF THE INVENTION

The present inventors have found that the expression level of mRNA of a certain group of genes decreased and concomitantly so did proteins encoded by the genes, when a pladienolide derivative was contacted with a sample obtained from living cells including cancerous cells and peripheral blood (PBMC) and whole blood (PBC) of a subject. Without wishing to be bound by any theory, administration of the pladienolide derivative may suppress transcription and translation of a certain group of genes, thereby decreasing the expression level of the genes. The present invention was made based on such findings.

It is an object of the present invention to provide a method, a probe, a primer, an antibody, a reagent, and a kit for assaying an action of the pladienolide derivative on a living subject.

According to the present invention, there are provided inventions (1) to (19) as follows.

(1) A method for assaying an action of a compound represented by formula (I), a pharmaceutically acceptable salt thereof, or a solvate of them to a mammal, which comprises detecting a decrease in gene expression level caused by the compound represented by formula (I), the pharmaceutically acceptable salt thereof, or the solvate of them:

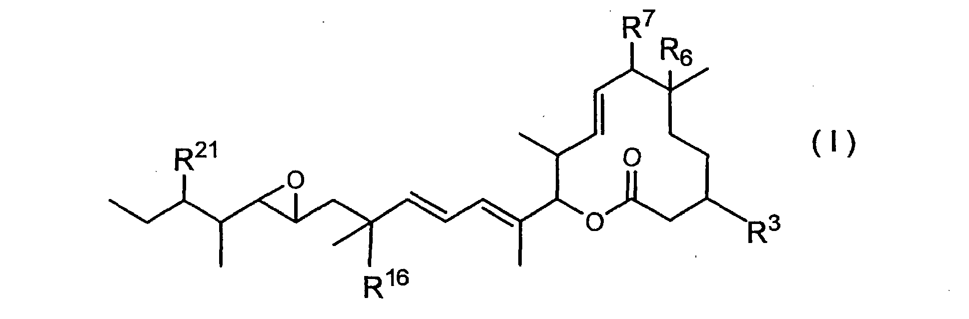

wherein R³ R⁶, R⁷ and R²¹, the same or different, each represents
1) a hydroxyl group or an oxo group formed together with the carbon atom to which it is bound, provided that R⁶ is limited to a hydroxyl group,
2) an optionally substituted C₁₋₂₂ alkoxy group,
3) an optionally substituted unsaturated C₂₋₂₂ alkoxy group,
4) an optionally substituted C₇₋₂₂ aralkyloxy group,
5) an optionally substituted 5- to 14-membered heteroaralkyloxy group,
6) RCO-O- wherein R represents
   a) a hydrogen atom,
   b) an optionally substituted C₁₋₂₂ alkyl group,
   c) an optionally substituted unsaturated C₂₋₂₂ alkyl group,
   d) an optionally substituted C₆₋₁₄ aryl group,
   e) an optionally substituted 5- to 14-membered heteroaryl group,
   f) an optionally substituted C₇₋₂₂ aralkyl group,
   g) an optionally substituted 5- to 14-membered heteroaralkyl group,
   h) an optionally substituted C₁₋₂₂ alkoxy group,
   i) an optionally substituted unsaturated C₂₋₂₂ alkoxy group,
   j) an optionally substituted C₆₋₁₄ aryloxy group or
   k) an optionally substituted 5- to 14-membered heteroaryloxy group,
7) R^{SI} R^{S2}R^{S3}SiO- wherein R^{SI}, R^{S2}, and R^{S3}, the same or different, each represents
   a) a C₁₋₆ alkyl group or
   b) a C₆₋₁₄ aryl group,
8) a halogen atom,
9) R^{N1}R^{N2}N-R^{M}- wherein R^{M} represents
   a) a single bond,
   b) -CO-O-,
   c) -SO₂-O-,
   d) -CS-O-or
   e) -CO-NR^{N3}- wherein R^{N3} represents a hydrogen atom or an optionally substituted C₁₋₆ alkyl group, provided that each of the leftmost bond in b) to e)is bound to the nitrogen atom; and R^{N1} and R^{N2}, the same or different from each other and each represents

   a) a hydrogen atom,
   b) an optionally substituted C₁₋₂₂ alkyl group,
   c) an optionally substituted unsaturated C₂₋₂₂ alkyl group,
   d) an optionally substituted aliphatic C₂₋₂₂ acyl group,
   e) an optionally substituted aromatic C₇₋₁₅ acyl group,
   f) an optionally substituted C₆₋₁₄ aryl group,
   g) an optionally substituted 5- to 14-membered heteroaryl group,
   h) an optionally substituted C₇₋₂₂ aralkyl group,
   i) an optionally substituted C₁₋₂₂ alkylsulfonyl group,
   j) an optionally substituted C₆₋₁₄ arylsulfonyl group,
   k) an optionally substituted 3- to 14-membered non-aromatic heterocyclic group formed by R^{NI} and R^{N2} together with the nitrogen atom to which R^{NI} and R^{N2} are bound, and the non-aromatic heterocyclic group optionally has substituent(s),
   l) an optionally substituted 5- to 14-membered heteroaralkyl group,
   m) an optionally substituted C₃₋₁₄ cycloalkyl group or
   n) an optionally substituted 3- to 14-membered non-aromatic heterocyclic group,
10) R^{N4}SO₂-O- wherein R^{N4} represents
   a) an optionally substituted C₁₋₂₂ alkyl group,
   b) an optionally substituted C₆₋₁₄ aryl group,
   c) an optionally substituted C₁₋₂₂ alkoxy group,
   d) an optionally substituted unsaturated C₂₋₂₂ alkoxy group,
   e) an optionally substituted C₆₋₁₄ aryloxy group,
   f) an optionally substituted 5- to 14-membered heteroaryloxy group,
   g) an optionally substituted C₇₋₂₂ aralkyloxy group or
   h) an optionally substituted 5- to 14-membered heteroaralkyloxy group,
11) (R^{N5}O)₂PO-O- wherein R^{N5} represents
   a) an optionally substituted C₁₋₂₂ alkyl group,
   b) an optionally substituted unsaturated C₂₋₂₂ alkyl group,
   c) an optionally substituted C₆₋₁₄ aryl group,
   d) an optionally substituted 5- to 14-membered heteroaryl group,
   e) an optionally substituted C₇₋₂₂ aralkyl group or
   f) an optionally substituted 5- to 14-membered heteroaralkyl group),
12) (R^{N1}R^{N2}N)₂PO-O- wherein R^{NI} and R^{N2} have the same meanings as defined above or
13) (R^{N1}R^{N2}N)(R^{N5}O)PO-O- wherein R^{N1}, R^{N2} and R^{N5} have the same meanings as defined above, provided that a compound in which R³, R⁶ ,R⁷ and R²¹ are all hydroxyl groups, and a compound in which R³, R⁶ and R²¹ are all hydroxyl groups and R⁷ is an acetoxy group are excluded,
   R¹⁶ represents a hydrogen atom or hydroxyl group. (2) The method according to (1), wherein the compound represented by formula (I) is selected from the group consisting of:
   (8E,12E,14E)-7-(N-(2-(N',N'-Dimethylamino)ethyl)-N-methytcar bamoyloxy)-3,6,16,21-tetrahydroxy-6,10,12,16,20-pentamethyl -18,19-epoxytricosa-8,12,14-trien-11-olide;
   (8E,12E,14E)-3,6,16,21-Tetrahydroxy-6,10,12,16,20-pentameth yl-7-((4-methylhomopiperazin-1-yl)carbonyl)oxy-18,19-epoxytri cosa-8,12,14-trien-11-olide;
   (8E,12E,14E)-3,6,16,21-Tetrahydroxy-6,10,12,16,20-pentameth yl-7-((4-methylpiper-azin-1-yl)carbonyl)oxy-18,19-epoxytricosa-8,12,14-trien-11-olid e;
   (8E,12E,14E)-7-((4-Butylpiperazin-1-yl)carbonyl)oxy-3,6,16,21-tetrahydroxy-6,10,12,16,20-pentamethyl-18,19-epoxytricosa-8, 12,14-trien-11-olide;
   (8E,12E,14E)-7-((4-Ethylpiperazin-1-yl)carbonyl)oxy-3,6,16,21-tetrahydroxy-6,10,12,16,20-pentamethyl-18,19-epoxytricosa-8, 12,14-trien-li-olide;
   (8E,12E,14E)-3,6,16,21-Tetrahydroxy-6,10,12,16,20-pentameth yl-7-((4-propylpiperazin-1-yl)carbonyl)oxy-18,19-epoxytricosa-8,12,14-trien-11-olide;
   (8E,12E,14E)-7-((4-Cyclohexylpiperazin-1-yl)carbonyl)oxy-3,6,1 6,21-tetrahydroxy-6,10,12,16,20-pentamethyl-18,19-epoxytrico sa-8,12,14-trien-11-olide;
   (8E,12E,14E)-7-((4-(Cyclopropylmethyl)piperazin-1-yl)carbonyl) oxy-3,6,16,
   21-tetrahydroxy-6,10,12,16,20-pentamethyl-18,19-epoxytricosa -8,12,14-trien-11-olide;
   (8E,12E,14E)-3,6,16,21-Tetrahydroxy-6,10,12,16,20-pentameth yl-7-((4-propylhomopiperazin-1-yl)carbonyl)oxy-18,19-epoxytric osa-8,12,14-trien-11-olide;
   (8E,12E,14E)-7-((4-(Cyclopropylmethyl)homopiperazin-1-yl)car bonyl)oxy-3,6,
   16,21-tetrahydroxy-6,10,12,16,20-pentamethyl-18,19-epoxytric osa-8,12,14-trien-11-olide;
   (8E,12E,14E)-7-((4-Cyclopentylpiperazin-1-yl)carbonyl)oxy-3,6, 16,21-tetrahydroxy-6,10,12,16,20-pentamethyl-18,19-epoxytric osa-8,12,14-trien-11-olide;
   (8E,12E,14E)-3,6,16,21-Tetrahydroxy-7-((4-isopropylpiperazin-1-yl)carbonyl)oxy-6,10,12,16,20-pentamethyl-18,19-epoxytrico sa-8,12,14-trien-11-olide;
   (8E,12E,14E)-7-((4-Cycloheptylpiperazin-1-yl)carbonyl)oxy-3,6, 16,21-Tetrahydroxy-6,10,12,16,20-pentamethyl-18,19-epoxytric osa-8,12,14-trien-11-olide;
   (8E,12E,14E)-7-(N-(2-(N', N'-Diethylamino)ethyl)-N-methylcarba moyloxy)-3,6,16,21-tetrahydroxy-6,10,12,16,20-pentamethyl-1 8,19-epoxytricosa-8,12,14-trien-11-olide;
   (8E,12E,14E)-3,6,16,21-Tetrahydroxy-7-((4-isobutylhomopipera zin-1-yl)carbonyl)oxy-6,10,12,16,20-pentamethyl-18,19-epoxyt ricosa-8,12,14-trien-11-olide;
   (8E,12E,14E)-7-((4-Ethylhomopiperazin-1-yl )carbonyl)oxy-3,6,1 6,21-tetrahydroxy-6,10,12,16,20-pentamethyl-18,19-epoxytrico sa-8,12,14-trien-11-olide;
   (8E,12E,14E)-7-((4-Butylhomopiperazin-1-yl)carbonyl)oxy-3,6,1 6,21-tetrahydroxy-6,10,12,16,20-pentamethyl-18,19-epoxytrico sa-8,12,14-trien-11-olide;
   (8E,12E,14E)-3,16,21-Trihydroxy-6-methoxy-6,10,12,16,20-pen tamethyl-7-((4-methylpiperazin-1-yl)carbonyl)oxy-18,19-epoxyt ricosa-8,12,14-trien-11-olide;
   (8E,12E,14E)-3,16,21-Trihydroxy-6-methoxy-6,10,12,16,20-pen tamethyl-7-((4-(piperidin-1-yl)piperidin-1-yl)carbonyl)oxy-18,1 9-epoxytricosa-8,12,14-trien-11-olide;
   (8E,12E,14E)-3,6,7,21-Tetrahydroxy-6,10,12,16,20-pentamethyl -18,19-epoxytricosa-8,12,14-trien-11-olide;
   (8E,12E,14E)-7-((4-(2,2-Dimethylpropyl)homopiperazin-1-yl)car bonyl)oxy-3,6,16,21-tetrahydroxy-6,10,12,16,20-pentamethyl-1 8,19-epoxytricosa-8,12,14-trien-11-olide; and
   (8E,12E,14E)-3,6,16-Trihydroxy-21-methoxy-6,10,12,16,20-pen tamethyl-7-((4-methylpiperazin-1-yl)carbonyl)oxy-18,19-epoxyt ricosa-8,12,14-trien-11-olide.

(3) The method according to (1), wherein the detection of the decrease in the gene expression level comprises the steps of:
(a) measuring the expression level of mRNA before and after administration of the compound represented by formula (I), the pharmaceutically acceptable salt thereof ,or the solvate of them to a mammal;
(b) comparing, based on the expression level measured in (a), the expression level of the mRNA before and after administration of the compound represented by formula (I), the pharmaceutically acceptable salt thereof, or the solvate of them, to determine that the compound represented by formula (I), the pharmaceutically acceptable salt thereof, or the solvate of them exerts an action to the mammal when the expression level of mRNA after the administration decreases.

(4) The method according to (3), wherein the mRNA of which expression level is measured is mRNA of at least one gene selected from the genes listed in Table 1, Table 2, Table 3 and Table 4, or a homologous gene thereof.

(5) The method according to (4), wherein the gene(s) are selected from TRAPPC4, SLC25A19, GTF2H1, ID1, ZCCHC6 and EDN1.

(6) The method according to (3), wherein in step (a), the expression level of mRNA in samples obtained from a subject before and after administration of the compound represented by formula (I), the pharmaceutically acceptable salt thereof, or the solvate of them, is measured.

(7) The method according to (6), wherein the samples obtained from the subject are selected from hemocytes in peripheral blood, plasma and serum.

(8) A probe or primer for assaying an action of a compound represented by formula (I), a pharmaceutically acceptable salt thereof, or a solvate of them, which consists of a polynucleotide capable of hybridizing with a polynucleotide consisting of a nucleotide sequence of at least one gene selected from the genes listed in Table 1, Table 2, Table 3 and Table 4, or a homologous gene thereof, or a complementary sequence thereof.

(9) The probe or primer according to (8), which is capable of detecting a genomic intron region or a part thereof in a gene listed in Table 1, Table 2, Table 3 or Table 4, or which is capable of detecting a polynucleotide lacking a part of a genomic exon region in a gene listed in Table 1, Table 2, Table 3 or Table 4.

(10) A reagent or kit for assaying an action of a compound represented by formula (I), a pharmaceutically acceptable salt thereof, or a solvate of them to a mammal, which comprises the probe or the primer according to (8).

(11) The method according to (1), wherein the detection of the decrease in the gene expression level comprises the steps of:
(f) measuring the expression level of a protein before and after administration of the compound represented by formula (I), the pharmaceutically acceptable salt thereof, or the solvate of them to a mammal;
(g) comparing, based on the expression level measured in (f), the expression level of the protein before and after administration of the compound represented by formula (I), the pharmaceutically acceptable salt thereof or the solvate of them, to determine that the compound represented by formula (I), the pharmaceutically acceptable salt thereof, or the solvate of them exerts an action to the mammal when the expression level of the protein after the administration decreases.

(12) The method according to (11), wherein the protein of which expression level is measured is a protein consisting of amino acids encoded by a polynucleotide of at least one gene selected from the genes listed in Table 1, Table 2, Table 3 and Table 4 or a homologous gene thereof.

(13) The method according to (11), wherein the protein of which expression level is measured is a protein consisting of amino acids encoded by a polynucleotide of at least one gene selected from TRAPPC4, SLC25A19, GTF2H1, ID1, ZCCHC6 and EDN1.

(14) The method according to (11), wherein in step (f), the expression level of the protein in the samples obtained from a subject before and after administration of the compound represented by formula (I) or the pharmaceutically acceptable salt thereof or the solvate of them, is measured.

(15) The method according to (14), wherein the samples obtained from the subject are selected from hemocytes in peripheral blood, plasma and serum.

(16) An antibody against an protein consisting of amino acids encoded by a polynucleotide of at least one gene selected from the genes listed in Table 1, Table 2, Table 3 and Table 4 or a homologous gene thereof, or a fragment thereof.

(17) A reagent or kit for assaying an action of a compound represented by formula (I), a pharmaceutically acceptable salt thereof, or a solvate of them to a mammal, comprising the antibody or the fragment thereof according to (16).

(18) Use of a probe or primer consisting of a polynucleotide capable of hybridizing with a polynucleotide consisting of a nucleotide sequence of at least one gene selected from the genes listed in Table 1, Table 2, Table 3, and Table 4, or a homologous gene thereof, or a complementary sequence thereof, for assaying an action of a compound represented by formula (I), a pharmaceutically acceptable salt thereof, or a solvate of them to a mammal.

(19) Use of an antibody against a protein consisting of amino acids encoded by at least one gene selected from the genes listed in Table 1, Table 2, Table 3, and Table 4, or a homologous gene thereof, or a fragment of the antibody, for assaying an action of a compound represented by formula (I), a pharmaceutically acceptable salt thereof, or a solvate of them to a mammal.

According to the present invention, an action of the compound represented by formula (I) to a living body can be confirmed using the expression level of a gene in a cancerous and normal tissue of a cancer patient or a normal tissue of a healthy individual, more specifically, the expression level of mRNA or the expression level of a protein as an index. For instance, when the expression level of the gene decreases in the cancerous or normal tissue of the cancer patient, more specifically the expression level of mRNA or the expression level of the protein decreases, it can be determined that the compound represented by formula (I) exerts the action and that thus administration of the drug is no longer required or less amount of the drug should be administrated. Further, when the expression level of the gene does not exhibit a downtrend in the cancerous or normal tissue of the cancer patient, more specifically, the expression level of mRNA or the expression level of the protein exhibits an uptrend or substantially reach the same amount as before the administration, it can be determined that the compound represented by formula (I) does not exert the action and further administration of the drug is required. Hence, according to the invention, by monitoring periodically the expression of mRNA or the expression level of the protein, the antitumor agent can be administered more effectively to the patient and a minimally required amount of the drug can be administered to the patient. In particular, since the action of the compound represented by formula (I) can be judged by monitoring the expression level of mRNA and the expression level of the protein in samples obtained from the normal tissue such as blood of the patient, it is an advantage that the action of the compound represented by formula (I) to the living body can be readily and reliably assessed.

### BRIEF DESCRIPTION OF THE DRAWINGS

Figure 1 shows mRNA expression level. A: Results of the samples obtained from the Tempus Blood RNA Tube; B: Results of the samples obtained from the PAXgene Blood RNA Tube.
Figure 2 shows expression level of mRNA in whole blood (PBC) samples obtained from human peripheral blood. A: Results of the samples obtained from the Tempus Blood RNA Tube; B: Results of the samples obtained from the PAXgene Blood RNA Tube.
Figure 3 shows results measured expression of mRNA (TRAPPC4, SLC25A19, and GTF2H1) in blood of nude mice to which human colon carcinoma cells were subcutaneously transplanted and the pladienolide derivatives was administrated.
Figure 4 shows results measured expression of mRNA (TRAPPC4, SLC25A19, and GTF2H1) in tumor of nude mice to which human colon carcinoma cells were subcutaneously transplanted and the pladienolide derivatives was administrated.

### DETAILED DESCRIPTION OF THE INVENTION

All technical terms, scientific terms and terminologies used in the present specification have the same meanings as those that are generally understood by those ordinary skilled in the art in the technical fields to which the present invention belongs, and are used merely for the purpose of explaining a specific embodiment but are not intended to make limitation. The present invention can be carried out in various embodiments as long as not departing from the spirit thereof. All the prior art documents, published publications, patent publications and other patent documents cited in the present specification are incorporated into the present specification as references, and can be used for carrying out the present invention.

The "halogen atom" used in the specification of the present application means a fluorine atom, a chlorine atom, a bromine atom and an iodine atom. Among them, for example, a fluorine atom, a chlorine atom or a bromine atom is preferred, of which a fluorine atom or a chlorine atom is preferred.

The "C₁₋₂₂ alkyl group" used in the specification of the present application indicates a linear or branched alkyl group having 1 to 22 carbon atoms, such as methyl group, ethyl group, n-propyl group, iso-propyl group, n-butyl group, iso-butyl group, sec-butyl group, tert-butyl group, n-pentyl group, 1,1-dimethylpropyl group, 1,2-dimethylpropyl group, 2,2-dimethylpropyl group, 1-ethylpropyl group, n-hexyl group, 1-ethyl-2-methylpropyl group, 1,1,2-trimethylpropyl group, 1-propylpropyl group, 1-methylbutyl group, 2-methylbutyl group, 1,1-dimethylbutyl group, 1,2-dimethylbutyl group, 2,2-dimethylbutyl group, 1,3-dimethylbutyl group, 2,3-dimethylbutyl group, 2-ethylbutyl group, 2-methylpentyl group, 3-methylpentyl group, n-heptyl group, n-octyl group, n-nonyl group or n-decyl group; preferably a linear or branched alkyl group having 1 to 6 carbon atoms, such as methyl group, ethyl group, n-propyl group, iso-propyl group, n-butyl group, iso-butyl group, sec-butyl group, tert-butyl group or n-pentyl group; and more preferably, for example, methyl group, ethyl group, propyl group, iso-propyl group, n-butyl group, iso-butyl group or tert-butyl group.

The "unsaturated C₂₋₂₂ alkyl group" used in the specification of the present application indicates a linear or branched alkenyl group having 2 to 22 carbon atoms or a linear or branched alkynyl group having 2 to 22 carbon atoms, such as vinyl group, allyl group, 1-propenyl group, isopropenyl group, 2-methyl-1-propenyl group, 2-methyl-2-propenyl group, 1-butenyl group, 2-butenyl group, 3-butenyl group, 1-pentenyl group, 1-hexenyl group, 1,3-hexadienyl group, 1,5-hexadienyl group, ethynyl group, 1-propynyl group, 2-propynyl group, 1-butynyl group, 2-butynyl group, 3-butynyl group, 1-ethynyl-2-propynyl group, 2-methyl-3-butynyl group, 1-pentynyl group, 1-hexynyl group, 1,3-hexanediynyl group or 1,5-hexanediynyl group. It preferably indicates a linear or branched alkenyl group having 2 to 10 carbon atoms or a linear or branched alkynyl group having 2 to 10 carbon atoms, such as vinyl group, allyl group, 1-propenyl group, 2-propenyl group, isopropenyl group, 3-methyl-2-butenyl group, 3,7-dimethyl-2,6-octadienyl group, ethynyl group, 1-propynyl group, 2-propynyl group, 1-butynyl group, 2-butynyl group, 3-butynyl group or 3-methyl-1-propynyl group.

The "C₆₋₁₄ aryl group" used in the specification of the present application means an aromatic cyclic hydrocarbon group having 6 to 14 carbon atoms, and a monocyclic group and condensed rings such as a bicyclic group and a tricyclic group are included. Examples thereof include phenyl group, indenyl group, 1-naphthyl group, 2-naphthyl group, azulenyl group, heptalenyl group, indacenyl group, acenaphthyl group, fluorenyl group, phenalenyl group, phenanthrenyl group and anthracenyl group; and preferred examples include phenyl group, 1-naphthyl group and 2-naphthyl group.

The "5- to 14-membered heteroaryl group" used in the specification of the present application means a monocyclic, bicyclic or tricyclic 5- to 14-membered aromatic heterocyclic group which contains one or more of hetero atoms selected from the group consisting of nitrogen atom, sulfur atom and oxygen atom. Preferred examples thereof include nitrogen-containing aromatic heterocyclic groups such as pyrrolyl group, pyridyl group, pyridazinyl group, pyrimidinyl group, pyrazinyl group, triazolyl group, tetrazolyl group, benzotriazolyl group, pyrazolyl group, imidazolyl group, benzimidazolyl group, indolyl group, isoindolyl group, indolizinyl group, purinyl group, indazolyl group, quinolyl group, isoquinolyl group, quinolizinyl group, phthalazinyl group, naphthyridinyl group, quinoxalinyl group, quinazolinyl group, cinnolinyl group, pteridinyl group, imidazotriazinyl group, pyrazinopyridazinyl group, acridinyl group, phenanthridinyl group, carbazolyl group, carbazolinyl group, perimidinyl group, phenanthrolinyl group, phenazinyl group, imidazopyridinyl group, imidazopyrimidinyl group, pyrazolopyridinyl group and pyrazolopyridinyl group; sulfur-containing aromatic heterocyclic groups such as thienyl group and benzothienyl group; and oxygen-containing aromatic heterocyclic groups such as furyl group, pyranyl group, cyclopentapyranyl group, benzofuryl group and isobenzofuryl group; aromatic heterocyclic groups containing two or more different hetero atoms, such as thiazolyl group, isothiazolyl group, benzothiazolyl group, benzthiadiazolyl group, phenothiazinyl group, isoxazolyl group, furazanyl group, phenoxazinyl group, oxazolyl group, isoxazoyl group, benzoxazolyl group, oxadiazolyl group, pyrazolooxazolyl group, imidazothiazolyl group, thienofuranyl group, furopyrrolyl group and pyridoxazinyl group; and preferred examples include thienyl group, furyl group, pyridyl group, pyridazyl group, pyrimidyl group and pyrazyl group.

The "3- to 14-membered non-aromatic heterocyclic group" used in the specification of the present application indicates a monocyclic, bicyclic or tricyclic 3- to 14-membered non-aromatic heterocyclic group which may contain one or more hetero atoms selected from the group consisting of nitrogen atom, sulfur atom and oxygen atom. Preferred examples thereof include aziridinyl group, azetidinyl group, pyrrolidinyl group, pyrrolyl group, piperidinyl group, piperazinyl group, homopiperidinyl group, homopiperazinyl group, imidazolyl group, pyrazolidyl group, imidazolidyl group, morpholyl group, thiomorpholyl group, imidazolinyl group, oxazolinyl group, 2,5-diazabicyclo[2.2.1]heptyl group, 2,5-diazabicyclo[2.2.2]octyl group, 3,8-diazabicyclo[3.2.1]octyl group, 1,4-diazabicyclo[4.3.0]nonyl group, quinuclidyl group, tetrahydrofuran-yl group and tetrahydrothiophen-yl group. The non-aromatic heterocyclic groups also include groups derived from pyridone ring, and non-aromatic fused rings (e.g., a group derived from, for example, phthalimide ring or succinimide ring).

The "C₇₋₂₂ aralkyl group" used in the specification of the present application means a group corresponding to the above-defined "C₁₋₂₂ alkyl group" of which substitutable moiety is replaced by the above-defined "C₆₋₁₄ aryl group". Specific examples thereof include benzyl group, phenethyl group, 3-phenylpropyl group, 4-phenylbutyl group, 1-naphthylmethyl group and 2-naphthylmethyl group; and preferred examples include aralkyl groups having 7 to 10 carbon atoms such as benzyl group and phenethyl group.

The "5- to 14-membered heteroaralkyl group" used in the specification of the present application means a group corresponding to the above-defined "C₁₋₂₂ alkyl group" of which substitutable moiety is replaced by the above-defined "5- to 14-membered heteroaryl group". Specific examples thereof include thienylmethyl group, furylmethyl group, pyridylmethyl group, pyridazylmethyl group, pyrimidylmethyl group and pyrazylmethyl group; and preferred examples include thienylmethyl group, furylmethyl group and pyridylmethyl group.

The "C₃₋₁₄ cycloalkyl group" used in the specification of the present application indicates a cycloalkyl group having 3 to 14 carbon atoms, and suitable examples thereof include cyclopropyl group, cyclobutyl group, cyclopentyl group, cyclohexyl group, cycloheptyl group and cyclooctyl group; and preferred examples include cyclopentyl group, cyclohexyl group, cycloheptyl group and cyclooctyl group.

The "C₄₋₉ cycloalkyl alkyl group" used in the specification of the present application means a group corresponding to the above-defined "C₁₋₂₂ alkyl group" of which substitutable moiety is replaced by the above-defined "C₃₋₁₄ cycloalkyl group". Specific examples thereof include cyclopropylmethyl group, cyclobutylmethyl group, cyclopentylmethyl group, cyclohexylmethyl group, cycloheptylmethyl group and cyclooctylmethyl group; and preferred example include cyclopropylmethyl group, cyclobutylmethyl group and cyclopentylmethyl group.

The "C₁₋₂₂ alkoxy group" used in the specification of the present application means a group corresponding to the above-defined "C₁₋₂₂ alkyl group" to which end an oxygen atom is bonded. Suitable examples thereof include methoxy group, ethoxy group, n-propoxy group, iso-propoxy group, n-butoxy group, iso-butoxy group, sec-butoxy group, tert-butoxy group, n-pentyloxy group, iso-pentyloxy group, sec-pentyloxy group, n-hexoxy group, iso-hexoxy group, 1,1-dimethylpropyloxy group, 1,2-dimethylpropyloxy group, 2,2-dimethylpropoxy group, 1-methyl-2-ethylpropoxy group, 1-ethyl-2-methylpropoxy group, 1,1,2-trimethylpropoxy group, 1,2,2-trimethylpropoxy group, 1,1-dimethylbutoxy group, 1,2-dimethylbutoxy group, 2,2-dimethylbutoxy group, 2,3-dimethylbutyloxy group, 1,3-dimethylbutoxy group, 2-ethylbutoxy group, 2-methylpentoxy group, 3-methylpentyloxy group and hexyloxy group; and preferred examples include methoxy group, ethoxy group, n-propoxy group, iso-propoxy group, iso-butoxy group and 2,2-dimethylpropyloxy group.

The "unsaturated C₂₋₂₂ alkoxy group" used in the specification of the present application means a group corresponding to the above-defined "unsaturated C₂₋₂₂ alkyl group" to which end an oxygen atom is bonded. Suitable examples thereof include vinyloxy group, allyloxy group, 1-propenyloxy group, 2-propenyloxy group, isopropenyloxy group, 2-methyl-1-propenyloxy group, 2-methyl-2-propenyloxy group, 1-butenyloxy group, 2-butenyloxy group, 3-butenyloxy group, 1-pentenyloxy group, 1-hexenyloxy group, 1,3-hexadienyloxy group, 1,5-hexadienyloxy group, propargyloxy group and 2-butynyloxy group; and preferred examples include allyloxy group, propargyloxy group and 2-butynyloxy group.

The "C₆₋₁₄ aryloxy group" used in the specification of the present application means a group corresponding to the above-defined "C₆₋₁₄ aryl group" to which end an oxygen atom is bonded. Specific examples thereof include phenyloxy group, indenyloxy group, 1-naphthyloxy group, 2-naphthyloxy group, azulenyloxy group, heptalenyloxy group, indacenyloxy group, acenaphthyloxy group, fluorenyloxy group, phenalenyloxy group, phenanthrenyloxy group and anthracenyloxy group; and preferred examples include phenyloxy group, 1-naphthyloxy group and 2-naphthyloxy group.

The "C₇₋₂₂ aralkyloxy group" used in the specification of the present application means a group corresponding to the above-defined "C₇₋₂₂ aralkyl group" to which end an oxygen atom is bonded. Specific examples thereof include benzyloxy group, phenethyloxy group, 3-phenylpropyloxy group, 4-phenylbutyloxy group, 1-naphthylmethyloxy group and 2-naphthylmethyloxy group; and preferred examples include benzyloxy group.

The "5- to 14-membered heteroaralkyloxy group" used in the specification of the present application means a group corresponding to the above-defined "5- to 14-membered heteroaralkyl group" to which end an oxygen atom is bonded. Specific examples thereof include thienylmethyloxy group, furylmethyloxy group, pyridylmethyloxy group, pyridazylmethyloxy group, pyrimidylmethyloxy group and pyrazylmethyloxy group; and preferred examples include thienylmethyloxy group, furylmethyloxy group and pyridinylmethyloxy group.

The "5- to 14-membered heteroaryloxy group" used in the specification of the present application means a group corresponding to the above-defined "5- to 14-membered heteroaryl group" to which end an oxygen atom is bonded. Specific examples thereof include pyrrolyloxy group, pyridyloxy group, pyridazinyloxy group, pyrimidinyloxy group, pyrazinyloxy group, triazolyloxy group, tetrazolyloxy group, benzotriazolyloxy group, pyrazolyloxy group, imidazolyloxy group, benzimidazolyloxy group, indolyloxy group, isoindolyloxy group, indolizinyloxy group, purinyloxy group, indazolyloxy group, quinolyloxy group, isoquinolyloxy group, quinolizyloxy group, phthalazyloxy group, naphthyridinyloxy group, quinoxalyloxy group, quinazolinyloxy group, cinnolinyloxy group, pteridinyloxy group, imidazotriazinyloxy group, pyrazinopyridazinyloxy group, acridinyloxy group, phenanthridinyloxy group, carbazolyloxy group, carbazolinyloxy group, perimidinyloxy group, phenanthrolinyloxy group, phenazinyloxy group, imidazopyridinyloxy group, imidazopyrimidinyloxy group, pyrazolopyridinyloxy group, pyrazolopyridinyloxy group, thienyloxy group, benzothienyloxy group, furyloxy group, pyranyloxy group, cyclopentapyranyloxy group, benzofuryloxy group, isobenzofuryloxy group, thiazolyloxy group, isothiazolyloxy group, benzothiazolyloxy group, benzothiadiazolyloxy group, phenothiazinyloxy group, isoxazoyloxy group, furazanyloxy group, phenoxazinyloxy group, oxazolyloxy group, isoxazolyloxy group, benzoxazolyloxy group, oxadiazolyloxy group, pyrazolooxazolyloxy group, imidazothiazolyloxy group, thienofuranyloxy group, furopyrrolyloxy group and pyridoxazinyloxy group; and preferred examples include thienyloxy group, pyridyloxy group, pyrimidyloxy group and pyrazyloxy group.

The "aliphatic C₂₋₂₂ acyl group" used in the specification of the present application means a group corresponding to the above-defined "C₁₋₂₂ alkyl group" or "unsaturated C₂₋₂₂ alkyl group" to which end a carbonyl group is bonded. Examples thereof include acetyl group, propionyl group, butyryl group, iso-butyryl group, valeryl group, iso-valeryl group, pivaloyl group, caproyl group, decanoyl group, lauroyl group, myristoyl group, palmitoyl group, stearoyl group, arachidoyl group, acryloyl group, propiol group, crotonyl group, iso-crotonyl group, olenoyl group and linolenoyl group; and preferred examples include aliphatic acyl groups having 2 to 6 carbon atoms, such as acetyl group, propionyl group, butyryl group, iso-butyryl group and acryloyl group.

The "aromatic C₇₋₁₅ acyl group" used in the specification of the present application means a group corresponding to the above-defined "C₆₋₁₄ aryl group" or "5- to 14-membered heteroaryl group" to which end a carbonyl group is bonded. Examples thereof include benzoyl group, 1-naphthoyl group, 2-naphthoyl group, picolinoyl group, nicotinoyl group, isonicotinoyl group, furoyl group and thiophenecarbonyl group; and preferred examples include benzoyl group, picolinoyl group, nicotinoyl group and isonicotinoyl group.

The "C₁₋₂₂ alkylsulfonyl group" used in the specification of the present application means a group corresponding to the above-defined "C₁₋₂₂ alkyl group" to which a sulfonyl group is bound. Specific examples thereof include methylsulfonyl group, ethylsulfonyl group, n-propylsulfonyl group and iso-propylsulfonyl group; and preferred examples include methylsulfonyl group.

The "C₆₋₁₄ arylsulfonyl group" used in the specification of the present application means a group corresponding to the above-defined "C₆₋₁₄ aryl group" to which a sulfonyl group is bound. Specific examples thereof include benzenesulfonyl group, 1-naphthalenesulfonyl group and 2-naphthalenesulfonyl group; and preferred examples include benzenesulfonyl group.

The "aliphatic C₂₋₂₂ acyloxy group" used in the specification of the present application means a group corresponding to the above-defined "aliphatic C₂₋₂₂ acyl group" to which end an oxygen atom is bonded. Specific examples thereof include acetoxy group, propionyloxy group and acryloxy group; and preferred examples include acetoxy group and propionyloxy group.

The "C₂₋₂₂ alkoxycarbonyl group" used in the specification of the present application means a group corresponding to the above-defined "C₁₋₂₂ alkoxy group" to which end a carbonyl group is bonded. Examples thereof include methoxycarbonyl group, ethoxycarbonyl group, n-propoxycarbonyl group, iso-propoxycarbonyl group, n-butoxycarbonyl group, iso-butoxycarbonyl group, sec-butoxycarbonyl group and tert-butoxycarbonyl group; and preferred examples include ethoxycarbonyl group, iso-propoxycarbonyl group and tert-butoxycarbonyl group.

The "unsaturated C₃₋₂₂ alkoxycarbonyl group" used in the specification of the present application means a group corresponding to the above-defined "unsaturated C₂₋₂₂ alkoxy group" to which end a carbonyl group is bonded. Examples thereof include vinyloxycarbonyl group, allyloxycarbonyl group, 1-propenyloxycarbonyl group, isopropenyloxycarbonyl group, propargyloxycarbonyl group and 2-butynyloxycarbonyl group; and preferred examples include allyloxycarbonyl group.

The "C₁₋₂₂ alkylthio group" used in the specification of the present application means a group corresponding to the above-defined "C₁₋₂₂ alkyl group" to which end a sulfur atom is bonded. Examples thereof include methylthio group, ethylthio group, n-propylthio group and iso-propylthio group; and preferred examples include methylthio group or ethylthio group.

The "C₁₋₂₂ alkylsulfinyl group" used in the specification of the present application means a group corresponding to the above-defined "C₁₋₂₂ alkyl group" to which end a sulfinyl group is bonded. Examples thereof include methylsulfinyl group, ethylsulfinyl group, n-propylsulfinyl group and iso-propylsulfinyl group; and preferred examples include methanesulfinyl group or ethanesulfinyl group.

The "C₁₋₂₂ alkylsulfonyloxy group" used in the specification of the present application means a group corresponding to the above-defined "C₁₋₂₂ alkylsulfonyl group" to which end an oxygen atom is bonded. Examples thereof include methylsulfonyloxy group, ethylsulfonyloxy group, n-propylsulfonyloxy group and iso-propylsulfonyloxy group; and preferred examples include methylsulfonyloxy group.

The substituent of the phrase "an optionally substituted" used in the specification of the present application may be one or more groups selected from:
(1) a halogen atom,
(2) a hydroxyl group,
(3) a thiol group,
(4) a nitro group,
(5) a nitroso group,
(6) a cyano group,
(7) a carboxyl group,
(8) a hydroxysulfonyl group,
(9) a amino group,
(10) a C₁₋₂₂ alkyl group (for example, methyl group, ethyl group, n-propyl group, iso-propyl group, n-butyl group, iso-butyl group, sec-butyl group and tert-butyl group),
(11) an unsaturated C₂₋₂₂ alkyl group (for example, vinyl group, allyl group, 1-propenyl group, 2-propenyl group, isopropenyl group, ethynyl group, 1-propynyl group, 2-propynyl group, 1-butynyl group, 2-butynyl group and 3-butynyl group),
(12) a C₆₋₁₄ aryl group (for example, phenyl group, 1-naphthyl group and 2-naphthyl group),
(13) a 5- to 14-membered heteroaryl group (for example, thienyl group, furyl group, pyridyl group, pyridazyl group, pyrimidyl group and pyrazyl group),
(14) a 3- to 14-membered non-aromatic heterocyclic group (for example, aziridinyl group, azetidyl group, pyrrolidinyl group, pyrrolyl group, piperidinyl group, piperazinyl group, homopiperidinyl group, homopiperazinyl group, imidazolyl group, pyrazolidyl group, imidazolidyl group, morpholyl group, thiomorpholyl group, imidazolinyl group, oxazolinyl group and quinuclidyl group),
(15) a C₃₋₁₄ cycloalkyl group (for example, cyclopropyl group, cyclobutyl group, cyclopentyl group, cyclohexyl group, cycloheptyl group and cyclooctyl group),
(16) a C₁₋₂₂ alkoxy group (for example, methoxy group, ethoxy group, n-propoxy group, iso-propoxy group, sec-propoxy group, n-butoxy group, iso-butoxy group and tert-butoxy group),
(17) an unsaturated C₂₋₂₂ alkoxy group (for example, vinyloxy group, allyloxy group, 1-propenyloxy group, 2-propenyloxy group, isopropenyloxy group, ethynyloxy group, 1-propynyloxy group, 2-propynyloxy group, 1-butynyloxy group and 2-butynyloxy group),
(18) a C₆₋₁₄ aryloxy group (for example, phenyloxy group, 1-naphthyloxy group and 2-naphthyloxy group),
(19) a C₇₋₂₂ aralkyloxy group (for example, benzyloxy group, phenethyloxy group, 3-phenylpropyloxy group, 4-phenylbutyloxy group, 1-naphthylmethyloxy group and 2-naphthylmethyloxy group),
(20) a 5- to 14-membered heteroaralkyloxy group (for example, thienylmethyloxy group, furylmethyloxy group, pyridylmethyloxy group, pyridazylmethyloxy group, pyrimidylmethyloxy group and pyrazylmethyloxy group),
(21) a 5- to 14-membered heteroaryloxy group (for example, thienyloxy group, furyloxy group, pyridyloxy group, pyridazyloxy group, pyrimidyloxy group and pyrazyloxy group),
(22) an aliphatic C₂₋₂₂ acyl group (for example, acetyl group, propionyl group, butyryl group, iso-butyryl group, valeryl group, iso-valeryl group, pivalyl group, caproyl group, decanoyl group, lauroyl group, myristoyl group, palmitoyl group, stearoyl group, arachidoyl group, acryl group, propiol group, crotonyl group, iso-crotonyl group, olenoyl group and linolenoyl group),
(23) an aromatic C₇₋₁₅ acyl group (for example, benzoyl group, 1-naphthoyl group and 2-naphthoyl group),
(24) an aliphatic C₂₋₂₂ acyloxy group (for example, acetoxy group, propionyloxy group and acryloxy group),
(25) a C₂₋₂₂ alkoxycarbonyl group (for example, methoxycarbonyl group, ethoxycarbonyl group, n-propoxycarbonyl group, iso-propoxycarbonyl group, n-butoxycarbonyl group, iso-butoxycarbonyl group, sec-butoxycarbonyl group and tert-butoxycarbonyl group),
(26) an unsaturated C₃₋₂₂ alkoxycarbonyl group (for example, vinyloxycarbonyl group, allyloxycarbonyl group, 1-propenyloxycarbonyl group, 2-propenyloxycarbonyl group, isopropenyloxycarbonyl group, propargyloxycarbonyl group and 2-butynyloxycarbonyl group),
(27) a C₁₋₂₂ alkylthio group (for example, methylthio group, ethylthio group, n-propylthio group and iso-propylthio group),
(28) a C₁₋₂₂ alkylsulfinyl group (for example, methylsulfinyl group, ethylsulfinyl group, n-propylsulfinyl group and iso-propylsulfinyl group),
(29) a C₁₋₂₂ alkylsulfonyl group (for example, methylsulfonyl group, ethaylsulfonyl group, n-propanesulfonyl group and iso-propanesulfonyl group),
(30) a C₆₋₁₄ arylsulfonyl group (for example, benzenesulfonyl group, 1-naphthalenesulfonyl group and 2-naphthalenesulfonyl group),
(31) a C₁₋₂₂ alkylsulfonyloxy group (for example, methylsulfonyloxy group, ethylsulfonyloxy group, n-propylsulfonyloxy group and iso-propanesulfonyloxy group),
(32) carbamoyl group, and
(33) formyl group.
   Among them, a preferred example is an amino group, a C₁₋₂₂ alkyl group, an unsaturated C₂₋₂₂ alkyl group, a C₆₋₁₄ aryl group, a 5- to 14-membered heteroaryl group, a 3- to 14-membered non-aromatic heterocyclic group and a C₃₋₁₄ cycloalkyl group, and a more preferred example is an amino group, a C₁₋₂₂ alkyl group, a 3- to 14-membered nonaromatic heterocyclic group and a C₃₋₁₄ cycloalkyl group. The above-mentioned (9) amino group and (31) carbamoyl group as the substituent in "an optionally substituted" may each be further substituted with one or two of a C₁₋₂₂ alkyl group, an unsaturated C₂₋₂₂ alkyl group or a C₅₋₁₄ aryl group.

In the present specification, the chemical formula of the compound according to the present invention is illustrated as a planimetric chemical formula for convenience but the compound can include certain isomers drawn from the chemical formula. The present invention can include all isomers and mixtures of the isomers such as a geometric isomer which is generated from the configuration of the compound, an optical isomer based on an asymmetric carbon, a rotamer, a stereoisomer and a tautomer. The present invention is not limited to the expediential description of the chemical formula, and can include either of isomers or a mixture thereof. Accordingly, when the compound according to the present invention has an asymmetric carbon in the molecule, and its optically active substance and racemate exist, any one is included. Further, when polymorphic crystals exist, the crystal form according to the present invention is not specifically limited to one form, and any one of the crystal forms may be single or a mixture of the crystal forms.

The "pharmaceutically acceptable salt" used in the present invention is not particularly restricted as long as it can form a salt with the compound represented by formula (I), and is pharmaceutically acceptable. Preferred examples thereof include halide hydroacid salt such as hydrochloric acid salt, hydrobromic acid salt, hydroiodic acid salt; inorganic acid salt such as sulphic acid salt, nitric acid salt, perchloric acid salt, phosphoric acid salt, carbonic acid salt, bicarbonic acid salt; organic carboxylic acid salt such as acetic acid salt, trifluoroacetic acid salt, maleic acid salt, tartaric acid salt, fumaric acid salt, citric acid salt; organic sulfonic acid salt such as methanesulfonic acid salt, trifluoro methanesulfonic acid salt, ethanesulfonic acid salt, benzenesulfonic acid salt, toluenesulfonic acid salt, camphorsulfonic acid salt; amino acid salt such as aspartic acid salt, glutamic acid salt; quaternary amine salt; alkaline metal salt such as sodium salt, potassium salt; and alkaline earth metal salt such as magnesium salt, calcium salt.

The "solvate" used in the present invention is not particularly restricted as long as it can form a solvate with the compound represented by formula (I) or the salt thereof, and is pharmaceutically acceptable. Preferred examples include hydrate, alcoholate such as ethanolate, and etherate.

The present invention also includes a metabolite generated by degradation of the compound represented by formula (I) within a living body, as well as a prodrug of the compound represented by formula (I) and the salt thereof. The "prodrug" used herein means an inert substance to which "an active moiety of a drug" (meaning "drug" corresponding to a prodrug) has been chemically modified, for the purpose of improvement of bioavailability and reduction of a side effect. After absorbed, it is metabolized into the active moiety *in vivo* and exerts an action. Accordingly, the term "prodrug" refers to any compound having a lower intrinsic activity than the corresponding "drug", which is, once administrated to a biological system, converted into the "drug" substance via a spontaneous chemical reaction, enzyme catalyzed reaction or metabolic reaction. Examples of such prodrugs include various prodrugs, for example, compounds produced by acylation, alkylation, phosphorylation, boration, carbonation, esterification, amidation, or urethanation of a functional group such as an amino, hydroxyl, or carboxyl group in the compound represented by formula (I). However, it should be noted that the exemplified prodrugs are not comprehensive but are merely typical, and other conventional various prodrugs can be prepared by a conventional method by a person having ordinary skill in the art from the compound represented by formula (I).

When the compound represented by formula (I) is administrated to a mammal in the method according to the present invention, the compound represented by formula (I) may be formulated by known methods. Conventional carriers are used for formulations, and the pharmaceutical products are prepared by conventional methods. Namely, when a solid formulation for oral use is prepared, a filler is added to the main drug, and if necessary, a binder, a disintegrant, a lubricant, a colorant, a flavoring agent and the like are added thereto, and then tablets, coated tablets, granules, powders, capsules and the like are prepared by conventional methods. It is needless to say that sugar coating, gelatin coating or suitable coating may be conducted on the tablet and granule, if necessary. When the compounds are formulated as an injection, a pH regulator, a buffer, a stabilizer, a solubilizer and the like are added to the main drug, if necessary, to prepare an subcutaneous, intramuscular, intra-articular or intravenous injection according to conventional procedures. When the compound represented by formula (I) is administered as a therapeutic or preventive agent for various diseases, it may be orally administered as tablets, powders, granules, capsules, syrups and the like, and may be parenterally administered as a spray, a suppository, an injection, a topical preparation or an infusion. Although the dose remarkably varies according to the severity of symptom, age, the kind of disease etc., approximately 1 mg to 100 mg per day for an adult is administered in general at one time or several times per day.

When the expression level of mRNA of the genes listed in Table 1, Table 2, Table 3, and Table 4 or homologous genes thereof and a protein encoded by the genes is monitored in the method according to the present invention, the expression level may be preferably monitored before administration of the compound represented by formula (I), followed by another monitoring at 3, 6, 8, 24, or 48 hours after the administration. In a preferred embodiment, follow-up monitoring of the expression level is carried out at the earliest three hours after the administration of the compound represented by formula (I).

Upon implementing the method according to the present invention the decrease in the gene expression level can be detected using a decrease in the expression level of mRNA or a decrease in the protein expression level associated with the decrease in the mRNA expression level as an index.

According to a first aspect of the invention, a method for assaying the action of the compound represented by formula (I), using the decrease in the expression level of mRNA as an index (invention (3)) is provided.

In step (a), cancer tissue or normal tissue such as hemocytes in peripheral blood, platelets, and serum can be taken from the mammal subjected to the assay and mRNA samples can be prepared from the obtained samples to quantify the expression level of mRNA. Preparation of mRNA is well known (for example, "Molecular Cloning, A Laboratory Manual 3d ed."(Cold Spring Harbor Press (2001)), and required devices, instruments, and reagents therefor are commercially available. Hence those skilled in the art may prepare mRNA with no difficulties using the devices, apparatuses, and reagents as needed.

Measurement of the expression level of mRNA in step (a) can be performed with any method selected from a Northern blot method, a dot blot method, an RT-PCR method, and a microarray (preferably, Human Genome U133 plus 2.0 array). The principles of these methods and how to carry out these methods are well-known, and the required devices and apparatuses therefor are commercially available. Moreover, in Examples below, an example in which the expression level of mRNA is measured with these methods will be described. Those skilled in the art can measure the expression level of mRNA with no difficulties using the Northern blot method, the dot blot method, the RT-PCR method, and the microarray. In the measurement of the expression level of mRNA in step (a), preferably, the microarray can be used.

For the measurement of the expression level of mRNA in step (a), a probe and a primer which consist of a polynucleotide capable of hybridizing with nucleotide sequences of genes listed in Table 1, Table 2, Table 3, and Table 4 and homologous genes thereof or their complementary sequences can be employed as a detection marker.

Any probe and primer according to the present invention may be employed as long as it can detect the expression of mRNA (including a part thereof) of the genes listed in Table 1, Table 2, Table 3, and Table 4 or homologous genes thereof. The probe and the primer according to the present invention refers to a polymer consisting of bases or base pairs such as deoxyribonucleic acid (DNA) or ribonucleic acid (RNA). It has been known that double stranded cDNA can be used in tissue in situ hybridization, and the probe and the primer according to the present invention include such double stranded cDNA. Particularly preferred RNA probe and primer for detecting RNA in tissue include RNA probes (riboprobe).

The probe and the primer according to the present invention includes a probe or a primer which consists of a polynucleotide comprising a nucleotide sequence of at least 10, preferably at least 15, more preferably at least 20, further preferably at least 25 continuous nucleotides, of the genes listed in Table 1, Table 2, Table 3, and Table 4, and homologous genes thereof, or complementary sequences thereof as well as all mutated polynucleotide sequences thereof. The probe and the primer according to the present invention include a probe or a primer which consists of a polynucleotide comprising a nucleotide sequence of 10 to 50 or 10 to 30 continuous nucleotides, 15 to 50 or 15 to 30 continuous nucleotides, 20 to 50 or 20 to 30 continuous nucleotides, 25 to 50 or 25 to 30 continuous nucleotides, of the genes listed in Table 1, Table 2, Table 3, and Table 4, and homologous genes thereof or mutated polynucleotide sequence thereof.

The probe and the primer according to the present invention may be at least 10 bases in length, preferably at least 15 bases in length, more preferably at least 20 bases in length, further preferably at least 25 bases in length. The probe and the primer according to the present invention may also be 10 to 50 bases or 10 to 30 bases in length, 15 to 50 bases or 15 to 30 bases in length, 20 to 50 bases or 20 to 30 bases in length, 25 to 50 bases or 25 to 30 bases in length.

According to the preferred embodiment of the probe and the primer according to the present invention, there are provided the probe and the primer having 15 to 30 bases in length for assaying the action of the compound represented by formula (I) to the mammals, which consist of a polynucleotide comprising at least 10, preferably at least 15, more preferably at least 20, further preferably at least 25 continuous nucleotides of a polynucleotide sequence of the genes listed in Table 1, Table 2, Table 3, and Table 4 or homologous genes thereof, as well as all mutated sequences thereof, and which are capable of hybridizing with a polynucleotide sequence of the genes listed in Table 1, Table 2, Table 3, and Table 4 or homologous genes thereof.

According to a preferred aspect of the probe and the primer according to the present invention, there are provided a probe and a primer which are capable of hybridizing with a more distinct region within a nucleotide sequence of the genes listed in Table 1, Table 2, Table 3, and Table 4 or homologous genes thereof. The above probe and primer allow more precise detection of the action of the compound represented by formula (I) to the mammal.

The probe according to the present invention can be chemically synthesized based on the nucleotide sequence of the gene subjected to be detected. Preparation of the probes is well known and can be carried out in accordance with, for example, "Molecular Cloning, A Laboratory Manual 2nd ed." (Cold Spring Harbor Press (1989)), "Current Protocols in Molecular Biology" (John Wiley & Sons (1987-1997)).

The primers according to the present invention can be used as a primer set comprising of two or more types of the primers.

The primer and the primer set according to the present invention can be used as a primer and a primer set in accordance with a conventional method in a known method for detecting the target gene using a nucleic acid amplification method such as a PCR method, a RT-PCR method, a real-time PCR method, and an *in situ* PCR method.

The primer set according to the present invention can be selected such that the nucleotide sequence of the target gene can be amplified with the nucleic acid amplification method such as the PCR method. The nucleic acid amplification method is well known and selection of the primer pair therein is obvious for those skilled in the art. For example, in the PCR method, the primers can be selected such that one of two primers (a primer pair) undergoes base pairing with the plus strand of the double stranded DNA of the gene subjected to be detected whereas the other of the primers undergoes base pairing with the minus strand of the double stranded DNA, as well as an extending strand extended with one primer can be paired with the other primer. In a LAMP method (WO00/28082), three regions from the 3' terminus termed F3c, F2c and F1c, and three regions from the 5' terminus termed B1, B2 and B3 are respectively defined for the gene subjected to be detected. Four types of primers can be designed using these six regions.

The primer according to the present invention may be chemically synthesized based on the nucleotide sequence of the gene subjected to be detected. Preparation of the primer is well known and can be carried out in accordance with, for example, "Molecular Cloning, A Laboratory Manual 2nd ed." (Cold Spring Harbor Press (1989)), "Current Protocols in Molecular Biology" (John Wiley & Sons (1987-1997)).

Table 1, Table 2, Table 3, and Table 4 describe information specifying the genes and homologous genes thereof listed in these tables. Accordingly those skilled in the art can obtain information on the nucleotide sequence of the subject gene to be detected based on the information described in Table 1, Table 2, Table 3, and Table 4 to design the probe and primer based thereon.

In addition, the genes listed in Table 1 and Table 2 are known genes and probes and primers for detecting them are commercially available individually or as a detection kit or detection array.

The term "to hybridize" used in the specification of the present application means to hybridize with a target polynucleotide under stringent conditions. A specific example of the polynucleotide which hybridizes under stringent conditions includes a polynucleotide having at least 70% or more, preferably 80% or more, more preferably 85% or more, further preferably 90% or more, further more preferably 95% or more, particularly preferably 98% or more, and most preferably 99% or more homology to the target polynucleotide when the homology is calculated by a homology search software, such as FASTA, BLAST, Smith-Waterman (Meth. Enzym., 164, 765, (1988)), using default parameters. Further, hybridization "under stringent conditions" can be performed, for example, by a method of carrying out the reaction at a temperature of 40°C to 70°C, preferably at a temperature of 60°C to 65°C, in a hybridization buffer solution generally used by those skilled in the art, and carrying out washing in a washing solution at a salt concentration of 15 to 300 mmol/L, preferably at 15 to 60 mmol/L. The temperature and salt concentration can be appropriately adjusted depending on the length of the probe to be used. Further the hybridized product can be washed under conditions in 0.2x or 2x SSC and 0.1% SDS at a temperature of 20°C to 68°C. Whether stringent (high stringency) or mild (low stringency) conditions is used depends on a difference in salt concentrations and temperatures while the washing process. In cases where the difference in hybridizing depends on the salt concentration, the washing process can be carried out in 0.2x SSC and 0.1% SDS as a stringent washing buffer (high stringency wash buffer) and 2x SSC and 0.1% SDS as a mild washing buffer (low stringency wash buffer). Also, in cases where the difference in hybridizing depends on the temperature, the washing process may be carried out at 68°C for stringent conditions, at 42°C for medium (moderate stringency) conditions, or at room temperature (20-25°C) for mild conditions, but 0.2x SSC and 0.1% SDS are used in all the cases.

When pre-hybridization is carried out, it is carried out under the same condition as in hybridization, but pre (preliminary)-washing is not necessarily carried out under the same condition as in hybridization.

The "homologous gene" used in the specification of the present application means a gene encoding a protein functionally equivalent to a protein encoded by a certain gene. Whether it is "functionally equivalent" or not can be determined by evaluating if the protein has functions equivalent to biological phenomena or functions related to the expression of the gene. Such a gene encoding the functionally equivalent protein includes not only the so-called homologous gene but also a gene with polymorphism and a gene having a mutation without affecting the function.

Examples of the homologous gene include genes which have a nucleotide sequence of a certain gene wherein one or more (preferably one to several, or 1, 2, 3 or 4) nucleotides are inserted, substituted or deleted, or added to one or both termini, and which encode the functionally equivalent protein.

Examples of the homologous gene also include genes which encode an amino acid sequence encoded by a certain gene wherein one or more amino acids are inserted, substituted, or deleted, or added to one or both termini (modified amino acid sequence), and which encode the functionally equivalent protein.

"One or more amino acids are inserted, substituted, or deleted, or added to one or both termini" used in the specification of the present application means that a modification is made by a known technical method such as a site specific mutagenesis method or by substitution of several amino acids as in naturally occurring mutation.

The "modified amino acid sequence" used in the specification of the present application can be an amino acid sequence wherein, for example, 1 to 30 amino acids, preferably 1 to 20 amino acids, more preferably 1 to 9 amino acids, further preferably 1 to 5 amino acids, particularly preferably 1 to 2 amino acids have been inserted, substituted, or deleted, or added to one or both termini. Preferably, the modified amino acid sequence may be an amino acid sequence having one or more (preferably one or several or 1, 2, 3, or 4) conservative substitutions.

The term "conservative substitution" is used herein to mean that one or more amino acid residues are substituted with other chemically similar amino acid residues, so as not to substantially modify the functions of a protein. Examples of such conservative substitution include a case where a certain hydrophobic residue is substituted with another hydrophobic residue and a case where a certain polar residue is substituted with another polar residue having the same electric charge. Such functionally similar amino acids that can be used in such substitution are known as every amino acid types in the present technical field. Specific examples of a nonpolar (hydrophobic) amino acid include alanine, valine, isoleucine, leucine, proline, tryptophan, phenylalanine, and methionine. Examples of a polar (neutral) amino acid include glycine, serine, threonine, tyrosine, glutamine, asparagine, and cysteine. Examples of a (basic) amino acid having a positive charge include arginine, histidine, and lysine. Examples of an (acidic) amino acid having a negative charge include aspartic acid and glutamic acid.

In cases where the first aspect of the present invention is carried out using a cancer tissue and its surrounding tissue as assay samples, the action of the compound represented by formula (I) can be assayed preferably by using the expression level of mRNA of the genes listed in Table 1 or homologous genes thereof as an index. Measurement of the expression level of the mRNA in cases where the cancer tissue and the surrounding tissue are used as the samples, the microarray can be preferably used.

In cases where the first aspect of the present invention is carried out using peripheral blood and whole blood as the assay samples, the action of the compound represented by formula (I) can be assayed preferably by using the expression level of mRNA of the genes listed in Table 2, Table 3, and Table 4, or homologous genes thereof as an index. Measurement of the expression level of the mRNA of the genes listed in Table 2, Table 3, and Table 4, or homologous genes thereof in cases where the peripheral blood and whole blood are used as the samples, the microarray can be preferably used.

According to a second aspect of the present invention, a method for assaying the action of the compound represented by formula (I), using a decrease in the expression level of a protein associated with a decrease in the mRNA expression level as an index (invention (11)) is provided.

The protein measured in step (f) is a protein encoded by the genes listed Table 1, Table 2, Table 3, and Table 4, or homologous genes thereof. Table 1, Table 2, Table 3, and Table 4 describe how to obtain sequence information of the genes listed in these tables.

In step (f), samples are taken from a cancer tissue or normal tissue such as hemocytes in peripheral blood, plasma, and serum from a mammal subjected to the assay. Measurement of the expression level of the protein may be carried out with the collected samples as they are or a protein extracted therefrom. Extraction of the protein is well known (for example, Campa, M.J. et al. Cancer Res. 63, 1652-1656, 2003), and devices, instruments, and reagents necessary for carrying out the extraction are commercially available. Hence those skilled in the art may extract the protein with no difficulties using such the commercially available devices, apparatuses, and reagents as needed.

The measurement of the expression level of the protein in step (f) can be carried out with a method selected from a fluorescent antibody method, an enzyme immunoassay (ELISA) method, a radioimmunoassay (RIA) method, a Western blot method and an immunostaining (immunohistochemistry) method. The principle and implementation procedures of these methods are well known and devices and instruments necessary for carrying out the methods are commercially available. Those skilled in the art may therefore measure the expression level of mRNA with no difficulties using the fluorescent antibody method, the enzyme immunoassay (ELISA) method, the radioimmunoassay (RIA) method, the Western blot method and the immunostaining (immunohistochemistry) method. In the measurement of the expression level of the protein in step (f), the enzyme immunoassay (ELISA) method, the Western blot method, the immunostaining (immunohistochemistry) method and a mass spectrometry method can be used.

In the measurement of the protein in step (f), an antibody against a protein encoded by the genes listed Table 1, Table 2, Table 3, and Table 4 and homologous genes thereof, and a fragment thereof can be used as a detection marker.

Any protein may be employed for obtaining the antibody according to the present invention as long as it has antigenicity. A protein having an amino acid sequence of the protein wherein one or more amino acids are deleted, inserted, substituted or added can be used as the antigen for the protein. It is known that such a protein maintains the same biological activity as the original protein (Mark et al. (1984) Proc.Natl.Acad.Sci. USA 81:5662-6; Zoller and Smith (1982) Nucleic Acids Res. 10:6487-500; Wang et al. (1984) Science 224:1431-3; Dalbadie-McFarland et al. (1982) Proc.NatLAcad.Sci.USA 79:6409-13). A technique to delete, insert, substitute or add one or more amino acids while maintaining the antigenicity of the original protein is known. For example, such a protein may be obtained by preparing and properly expressing a polynucleotide encoding a protein by site directed mutagenesis technique (Molecular Cloning, A Laboratory Manual 2nd ed., Cold Spring Harbor Press (1989); Current Protocols in Molecular Biology, John Wiley & Sons,(1987-1997),Section8.1-8.5; Hashimoto-Goto et al.(1995) Gene 152:271-5; Kinkel (1985) Proc.Natl.Acad.Sci.USA 82:488-92; Kramer and Fritz (1987) Method.Enzymol. 154:350-67; Kunkel (1988) Method.Enzymol. 85:2763-6).

The antibody according to the present invention includes an antibody having specificity against a part of the protein. That is, the protein for obtaining the antibody according to the present invention includes a polypeptide having the full length amino acid sequence of the protein as well as a fragment thereof having at least six amino acid residues (for example, not less than 6, 8, 10, 12 or 15 amino acid residues). A preferred fragment is a polypeptide fragment such as an amino terminus and a carboxyl terminus of the protein. An antigen determination site of the polypeptide can be predicted by a method analyzing the hydrophobicity/hydrophilicity of the amino acid sequence of the protein (Kyte-Doolittle (1982) J.Mol.Biol. 157:105-22), and a method analyzing a secondary structure (Chou-Fasman (1978) Ann.Rev.Biochem. 47:251-76) and further confirmed by a computer program (Anal.Biochem. 151:540-6 (1985)) or a technique such as PEPSCAN analysis (patent application publication JP60500684T) involving the synthesis of a short peptide to confirm the antigenicity.

Table 1, Table 2, Table 3, and Table 4 describe information specifying the genes listed in these Tables and homologous genes thereof. Accordingly, those skilled in the art can obtain information on an amino acid encoded by the subject gene to be detected based on the information described in Table 1, Table 2, Table 3, and Table 4, and can design and obtain an antibody based thereon.

In addition, the genes listed in Table 1, Table 2, Table 3, and Table 4 are known genes and an antibody for detecting a protein encoded thereby is commercially available individually or as a detection kit or detection array.

The antibody according to the present invention may be obtained with a method known those skilled in the art (for example, "Current Protocols in Molecular Biology" (John Wiley & Sons (1987), Antibodies: A Laboratory Manual, Ed. Harlow and David Lane, Cold Spring Harbor Laboratory (1988)).

The antibody according to the present invention includes a polyclonal antibody, a monoclonal antibody, a chimeric antibody, a single chain antibody (scFv), a humanized antibody and a multispecific antibody. Also, the fragment of the antibody according to the present invention includes an antibody fragment such as Fab, Fab', F(ab')₂, Fc, and Fv.

For a polyclonal antibody, blood can be taken from a mammal sensitized with an antigen and blood serum can be isolated with known procedures from the blood to yield blood serum containing the polyclonal antibody. As needed, a fraction containing the polyclonal antibody can further be isolated from this blood serum.

For a monoclonal antibody, antibody-producing cells are taken from spleen or lympho node of a mammal sensitized with the above-mentioned antigen, and then undergo cell fusion with myeloma cell. The resultant hybridoma is subjected to cloning and the antibody was recovered from the culture thereof to yield the monoclonal antibody.

A fragment of the protein can be used as an immunogen. Alternatively, the synthesized one based on the amino acid sequence of the protein can be used. The antigen can be used as a complex with a carrier protein. A variety of condensing agents can be used for preparation of the complex between the antigen and the carrier protein, which condensing agents include glutaraldehyde, carbodiimide, and maleimide active ester. The carrier protein may be a usually used one such as bovine serum albumin, thyroglobulin, and hemocyanin. A procedure for coupling at a rate (volume) of 1 time to 5 times is usually employed.

Examples of the animal immunized include mice, rats, rabbits, guinea pigs, hamsters. An example of a method of inoculation is subcutaneous, intramuscular or intraperitoneal administration. The administration may be done in combination with Freund's complete adjuvant and Freund's incomplete adjuvant, and usually once every two to five weeks.

The antibody-producing cells obtained from the spleen or lymph node of the animal immunized undergo cell fusion with myeloma cells, and is isolated as hybridoma. As the myeloma cells, cells derived from mouse, rat, Homo sapiens and etc. are used. It is preferred that antibody-producing cell be derived from the same species. Yet there are cases where the cell fusion can be carried out between different species.

Procedures for the cell fusion may be carried out with a known method, in accordance with, for example, Nature, 256,495, 1975. Examples of fusion accelerator include polyethylene glycols and Sendai virus. The cell fusion can be usually carried out by using about 20 to 50% of concentration of polyethylene glycols (average molecular weight 1000 to 4000); at a temperature of 20 to 40°C, preferably 30 to 37°C; at a ratio in number of cells between antibody production cells and myeloma of usually about 1:1 to 10:1, and for about 1 to 10 minutes.

Various immunochemical methods can be employed for screening the antibody-producing hybridoma. Examples thereof include ELISA method using a microtiter plate coated with the protein, EIA method using a microtiter plate coated with an anti-immunoglobulin antibody, immune blot method using a nitrocellulose blotting membrane after electrophoresis of samples containing the protein.

Using such wells, cloning by, for example, a limiting dilution method can be further carried out to obtain a clone. Selection and breeding of the hybridoma is usually carried out culture medium for mammalian cells (such as RPMI1640) containing 10∼20% bovine fetus serum and supplemented with HAT (hypoxanthine, aminopterin, and thymidine). The clone obtained in such a way is intraperitoneally transplanted into a SCID mouse previously administrated with pristine. Ten to fourteen days later, ascites containing the monoclonal antibody at a high concentration is obtained, which ascites can be used as a raw material for antibody purification. Also the clone may be cultured and the obtained culture may be used as a raw material for antibody purification

Any purification method may be used for purifying the monoclonal antibody as long as it is a known method for purifying an immunoglobulin. The purification can be readily accomplished by, for example, an ammonium sulfate fractionation method, a PEG fractionation method, an ethanol fractionation method, and use of an anion exchanger, as well as means such as affinity chromatography using the protein.

Purification of the polyclonal antibody from serum can be carried out in the same manner.

In cases where the procedure in the second aspect according to the present invention is carried out by using the cancer tissue and its surrounding tissue as assay samples, the action of the compound represented by formula (I) can preferably be assayed by using the expression level of the protein(s) encoded by the genes listed in Table1 or homologous genes thereof as an index. In cases where the cancer tissue and the surrounding tissue are used as the samples, the measurement of the expression level of the proteins can preferably be employed with the enzyme immunoassay (ELISA) method, the Western blot technique, the immunostaining (immunohistochemistry) method and the mass spectrometry method.

In cases where the procedure in the second aspect of the present invention using peripheral blood or whole blood as assay samples, the action of the compound represented by formula (I) can preferably be assayed by using the expression level of the protein(s) encoded by the genes listed in Table 2, Table 3, and Table 4 or homologous genes thereof as an index. In cases where peripheral blood or whole blood are used as the samples, the measurement of the expression level of the proteins can preferably be employed with the enzyme immunoassay (ELISA) method, the Western blot method, the immunostaining (immunohistochemistry) method and the mass spectrometry method.

The samples obtained from a subject refer to tissues, cells, body fluids, and the like which are obtained from the subject. Specific examples include biopsy, blood (including hemocytes, plasma, and serum), urine, tissue samples such as curettage tissue (buccal scrapes) of oral cavity, and tumor cells (cells from tumors of breast, lung, stomach, head and neck, colorectum, kidney, pancreas, uterus, liver, urinary bladder, endometrium, and prostate, as well as hemocytes of leukemia patients or of lymphocytes).

### EXAMPLES

The present invention is described in more detail by the examples below. The followings are illustrative of the invention and by no means intended to limit the invention to the embodiments described herein.

### Example 1: Analysis with microarray

Microarray analysis (Human Genome U133 plus 2.0 array: Affymetrix) was carried out using RNA purified from human colon carcinoma cell strain WiDr treated with 2.8 nM and 14 nM of (8E,12E,14E)-7-((4-Cycloheptylpiperazin-1-yl)carbonyl)oxy-3,6, 16,21-tetrahydroxy-6,10,12,16,20-pentamethyl-18,19-epoxytric osa-8,12,14-trien-11-olide (hereinafter also referred to as "compound A") for six hours,.

WiDr cells were first suspended in RPMI1640 medium (containing 10% FBS, penicillin, and streptomycin) and seeded in a 10 cm dish (2x10⁶ cells/dish). After an overnight culture in an incubator with 5% carbon dioxide gas at 37°C, the medium was changed with medium containing 2.8 nM and 14 nM of compound A or containing vehicle alone. After cultured additional six hours, TRI reagent (SIGMA) was added to cells, and the cells were harvested. RNA was purified in accordance with the protocol of TRI reagent, followed by further purification with RNeasy (QIAGEN). Absorbance at 260 nm was measured to quantify an amount of RNA.

Subsequently, single stranded cDNA synthesis, double stranded cDNA synthesis, biotin-labeled cRNA synthesis, and cRNA fragmentation in order were carried out from 5-10 µg (WiDr 10 µg, PBMC 5 µg) of total RNA from the control cells and the treated cells, by using One-Cycle Target Labeling and Control Reagents (Affymetrix). cRNA probe was used for hybridization with Human Genome U133 Plus 2.0 Array (Affymetrix). The array was then washed and stained, and luminescence intensity was measured by a scanner.

The RMA method was applied to 33 of .cel files obtained in the microarray experiment and log signal intensity at a gene level normalized with log signal intensity at a probe level was obtained. By subtracting the mean between control 1 and control 3 from the mean between log signal intensity 1 and log signal intensity 3 for each case, the log signal intensity was converted into a log signal ratio to the control. A t-test was conducted between each log signal intensity and control, and candidate genes were narrowed down in the basis of a p-value, a log signal ratio, and a signal value. Specifically, genes showing that the P value was 5% or less; the expression level in the untreated (control) is 100 or more by signal value; and the expression level when treated with both 2.8 nM and 14 nM of compound A is, compared to the control, 25% or less, were picked out. The software used is R2.2.1 (http://www.r-project.org/), affy package 1.8.1 (http://www.bioconductor.org).

Genes showing that, by the treatment with compound A, the P value was 5% or less; the expression level in the untreated (control) is 100 or more by signal value; and the expression level when treated with both 2.8 nM and 14 nM of compound A is, compared to the control, 25% or less, and results thereof were shown in Table 1. Probe ID used in Table 1 represents Human Genome U133 Plus 2.0 Array Probeset ID. In Table 1, for each gene evaluated, gene name (Gene Name), abbreviated name (Gene Symbol), accession number (Accession), alias name (Synonym), Probe set number in Human Genome U133 Plus 2.0 Array (Human Genome U133 Plus 2.0 Array Probeset ID), the expression level upon the six-hour treatment with 2.8 nM of E7107 (2.8 nM (6h)), and the expression level upon the six-hour treatment with 14 nM of E7107 (14 nM(6h)) were respectively shown.

**[Table 1]**

| Gene Name | Gene Symbol | Accession | Synonym | Human Genome U133 Plus 2.0 Array | 2.8 nM (6 h) | 14 nM (6 h) |
|---|---|---|---|---|---|---|
| | | | | Probeset ID | | |
| death-associated protein kinase 1 | DAPK1 | NM_004938 | DAPK // DKFZp781I035 | 203139_at | 0.09 | 0.07 |
| WD repeat domain 67 | WDR67 | NM_145647 | Gm85 // MGC104222 II MGC126773, // MGC138159 // MGC21654 | 214061_at | 0.09 | 0.08 |
| unc-50 homolog (C. elegans) | UNC50 | NM_014044 | DKFZp564G0222 // GMH1 // HSD23 // UNCL // URP // hGMH1p | 203583_at | 0.10 | 0.06 |
| fibroblast growth factor 19 | FGF19 | NM_005117 | - | 223761_at | 0.10 | 0.18 |
| Transcribed locus, moderately similar to XP_518244.1 | ___ | | | 235046-at t | 0.11 | 0.10 |
| MCM10 minichromosome maintenance deficient 10 (S. cerevisiae) | MCM10 | NM_018518 // NM_182751 | CNA43 // MGC126776 // PR02249 | 220651_s_at | 0.11 | 0.06 |
| hypothetical protein LOC51315 | LOC51315 | | | 218303_x_at | 0.11 | 0.07 |
| FKSG44 gene | FKSG44 | NM_031904 | FLJ32216 // MGC31785 | 227964 at | 0.11 | 0.06 |
| Iron-responsive element binding protein 2 | IREB2 | NM_004136 | //IRP2AD // IRP2AD | 225892_at | 0.12 | 0.03 |
| chromosome 7 open reading frame 23 | C7orf23 | NM_02431 | MGC4175 // MM-TRAG | 204215_at | 0.12 | 0.04 |
| nucleoporin like 1 | NUPL1 | NM_001008564 // NM_001008565 // NM_014089 | KIAA0410 // PR02463 | 223984_s_at | 0.12 | 0.12 |
| RAP1 interacting factor homolog (yeast) | RIF1 | NM_018151 | DKFZp781N1478 // | 226821_st | 0.13 | 0.07 |
| replication factor C (activator 1) 4, 37kDa | RFC4 | NM 002916 // NM_181573 | A1 // MGC27291 // RFC37 | 204023_at | 0.13 | 0.04 |
| tRNA splicing endonuclease 2 homolog (S. corevisiae) | TSEN2 | NM_025265 | MGC2776 // MGC4440 // SEN2 // SEN2L | 219581_at | 0.13 | 0.11 |
| CDC6 cell division cycle 6 homolog (S. cerevisiae) | CDC6 | NM_001254 | CDC18L // HsCDC18 // HsCD06 | 203967_at | 0.13 | 0.08 |
| Fanconi anemia, complementation group L | FANCL | NM_018062 | FLJ10335 // PHF9 // POG | 218397-at | 0.15 | 0.21 |
| jagunal homolog 1 (Drosophila) | JAGN1 | NM_032492 | FLJ14602 // GL009 | 223104_at | 0.15 | 0.09 |
| chromosome 1 open reading frame 63 | Ctorf63 | NM_020317 | DJ465N24.2.1 // NPD014 // RP3-465N24.4 | 209006_s_at | 0.15 | 0.19 |
| hypothetical protein LOC124512 | LOC124512 | // XM_497558 // XM_940962 // NM 001080510 | - | 225808-at | 0.15 | 0.16 |
| nuclear receptor subfamily 2, group C, member 1 | NR2C1 | NM_001032287 // NM-003297 | TR2 // TR2-11 | 204791-at | 0.15 | 0.13 |
| chromosome 1 open reading frame 79 | C1orf79 | XM_378848 // XM_930434 // XM_934809 //XM_934810 // XM_93481 // XM_940389 // XM_944565 // XM_944566 | RP4-669K10.5 | 223774_at | 0.16 | 0.14 |
| pleckstrin homology domain containing, family A member 5 | PLEKHA5 | NM_019012 | FLJ10667 // FLJ31492 // KIAA1686 // PEPP2 | 220952_s_at | 0.16 | 0.06 |
| EH domain binding protein 1 | EHBP1 | No 015252 | KIAA0903 // NACSIN | 212653_s_at | 0.16 | 0.05 |
| hypothetical protein FLJ20628 | FLJ20628 | NM 017910 | DKFZp56412178 | 221229_s_at | 0.16 | 0.25 |
| zinc finger, MIZ-type containing 1 | ZMIZ1 | NM_020338 | FLJ13541 // KIAAI 224 // MIZ // RAI17 // Zimp10 // hZIMP10 | 212124_at | 0.16 | 0.05 |
| polymerase (DNA-directed), delta 3, accessory subunit | POLD3 | NM_006591 | KIAA0039 // MGC119642 MGC119643 // P66 // P68 | // 212836_s_at | 0.16 | 0.09 |
| Transcribed locus, strongly similar to NP_689873.1 | __ | | | 228638 at | 0.16 | 0.12 |
| | | NM_001040184 // | | | | |
| | | NM_001040181 // | | | | |
| | | NM_001040182 // | C3orf4 // GENX-3745 // | | | |
| claudin domain containing 1 | CLDND1 | NM_001040183 // | MGC111162 // MGC3316 // | 208925_at | 0.16 | 0.09 |
| | | NM_001040199 // | MGC9861 | | | |
| | | NM_001040200 // | | | | |
| | | NM_019895 | | | | |
| chromosome 7 open reading frame 36 | C7orf36 | NM_020192 | GK003 | 223433_at | 0.16 | 0.15 |
| tubulin, gamma complex associated protein 3 | TUBGCP3 | NM_006322 | GCP3 // SPBC98 // Spc98p | 203690_at | 0.16 | 0.11 |
| KIAA0859 | KIAA0859 | NM_001007239 // NM_014955// NM_015935 | 5630401 D24Rik // CGI-01 // FLJ10310 | 212405_s_at | 0.17 | 0.09 |
| sideroflexin 1 | SFXN1 | NM_022754 | FLJ12876 | 230069_at | 0.17 | 0.06 |
| ATPase, H+ transporting, lysosomal V0 subunit a2 | ATP6VOA2 | NM_012463 | ATP6N1D // ATP6a2 // J6B7 // Stv1 // TJ6 // TJ6M // TJ6s // Vph1 // a2 | 205704_s_at | 0.17 | 0.15 |
| chromosome 15 open reading frame 40 | C15orf40 | NM_144597 | MGC29937 | 1552310_at | 0.17 | 0.19 |
| WW domain binding protein 4 (formin binding protein 21) | WBP4 | NM_007187 | FBP21 // MGC117310 | 203598_s_at | 0.17 | 0.17 |
| Transcribed locus | ___ | | | 230098 at | 0.17 | 0.13 |
| retinoblastoma binding protein 6 | RBBP6 | NM_006910 // NM_018703 //NM_032626 | DKFZp686P0638 // DKFZp761B2423 // MY038 P2P-R // RBQ-1 | 212783_at | 0.17 | 0.12 |
| meiotic nuclear divisions 1 homolog (S. cerevisiae) | MND1 | NM 032117 | GAJ | 223700-at | 0.17 | 0.14 |
| ecotropic viral integration site 1 | EVI1 | NM_005241 | AML1-EVI-1 // EVI-1 // MDS1-EVI1 // MGC163392 // PRDM3 | 226420_at | 0.17 | 0.07 |
| RNA binding motif, single stranded interacting protein 1 | RBMS1 | NM_002897 // NM_016836 // NM_016839 // | MGC15146 // MGC3331 // MSSP // MSSP-1 // MSSP-2 // MSSP-3 // SCR2 // | 209868_s_at | 0.17 | 0.11 |
| zinc finger, CCHC domain containing 6 | ZCCHC6 | NM_024617 | DKFZp666B142 // DKFZp686C11112 // DKFZp686F119 // DKFZp68611269 // PAPD6 | 220933_s_at | 0.18 | 0.25 |
| kinesin family member 21A | KIF21A | NM_017641 | CFEOM // CFEOM1 // DKFZp779C159 // FEOM // FEOM1 // FLJ20052 // KIAA1708 | 226003_at | 0.18 | 0.09 |
| polymerase (RNA) III (DNA directed) polypeptide E (80kD) | POLR3E | NM_018119 | RPC5 // SIN | 222490_at | 0.18 | 0.10 |
| synaptotagmin-like.2 | SYTL2 | NM_032379 // NM_032943 // 206928 // NM_206928 // NM_206929 // NM_206930 | CHR11SYT // KIAA1597 // MGC102768 // SGA72M // SLP2 | 232914_s_at | 0.18 | 0.24 |
| CDC7 cell division cycle 7 (S. cerevisiae) | CDC7 | NM_003503 | CDC7L1 // HsCDC7 // Hsk1 // MGC117361// MGC126237 // MGC126238 // huCDC7 | 204510_at | 0.18 | 0.13 |
| zinc finger protein 518 | ZNF518 | NM_014803 | DKFZp78102147 // MGC125707 // MGC125710 | 204291_at | 0.18 | 0.12 |
| Transcribed locus | ___ | | | 230177_at | 0.18 | 0.22 |
| KIAA0776 | KIAA0776 | NM_015323 | RP3-393D12.1 | 212633_at | 0.18 | 0.15 |
| | | XM_001126364 // | | | | |
| RAD54 homolog B (S. cerevisiae) | RAD54B | XM_001126393 // | FSBP | 219494_at | 0.18 | 0.10 |
| | | NM_012415 | | | | |
| chromosome 12 open reading frame 32 | C12orf32 | NM 031465 | HKMT1188 // MGC13204 | 225837_at | 0.19 | 0.17 |
| KIAA1387 protein | SMEK2 | NM_020463 | FLFL2 // FLJ31474 // KIAA1387 // PSY2 // smk1 | 226230_at | 0.19 | 0.14 |
| STAM binding protein-like 1 | STAMBPL1 | NM_020799 | ALMalpha // AMSH-FP // AMSH-LP // FLJ31524 // KIAA1373 // bA399O19.2 | 227606_s_at | 0.19 | 0.14 |
| SNF2 histone linker PHD RING helicase | SHPRH | NM_001042683 // NM_173082 | FLJ27258 // FLJ37625 // FLJ45012 // FLJ90837 // KIAA2023 // MGC134886 // bA545I5.2 | 226366_at | 0.19 | 0.08 |
| F-box and leucine-rich repeat protein 5 | FBXL5 | NM_012161 // NM_033535 | FBL4 // FBL5 // FLR1 | 209004 s at | 0.19 | 0.05 |
| syntaxin binding protein 3 | STXBP3 | XM_001129477 // XM_001131280 // NM_007269 | MUNC18-3 // MUNC18C // PSP // UNC-18C | 203310_at | 0.19 | 0.14 |
| family with sequence similarity 86, member C | FAM86C | NM_018172// NM_152563 | FLJ10661 // FLJ27199 // MGC45068 | 65585_at | 0.19 | 0.22 |
| bromodomain PHD finger transcription factor | BPTF | NM_004459 // NM_182641 | FAC1 // FALZ // NURF301 | 232909 s_at | 0.20 | 0.05 |
| ATPase, Ca++ transporting plasma membrane 1 | ATP2B1 | NM_001001323// NM_001682 // | PMCA1 | 212930_at | 0.20 | 0.10 |
| potassium voltage-gated channel, Isk-related family, member 3 | KCNE3 | NM_005472 | DKFZp781H21101 // HOKPP // MGC102685 // MGC129924 // MiRP2 | 227647_at | 0.20 | 0.16 |
| chromosome 7 open reading frame 49 | C7orf49 | NM_024033 | MGC5242 FLJ27285// | 220949_s_at | 0.20 | 0.23 |
| chromosome 1 open reading frame 82 | C1orf82 | NM_024813 | FLJ13150 // RP11-163M2.4 | 222893_s_at | 0.20 | 0.19 |
| kinesin family member 2C | KIF2C | NM_006845 | KNSL6 // MCAK | 211519_s_at | 0.20 | 0.12 |
| kelch-like 12 (Drosophila) | KLHL12 | NM_021633 | C31P1 // DKIR // FLJ27152 | 225068_at | 0.20 | 0.11 |
| tensin 3 | TNS3 | NM_022748 | DKFZp686K12123 // DKFZp686M1045 // FLJ13732 // FLJ35545 // H_NH049123.2 // MGC88434 // TEM6 // TENS1 | 217853_at | 0.20 | 0.07 |
| chromosome 5 open reading frame 28 | C5orf28 | NM_022483 | FLJ21657// MGC149524 MGC90226 | // 219029_at | 0.20 | 0.05 |
| myosin head domain containing 1 | MYOHD1 | NM_001033579 // NM_001033580 // NM 025109 | FLJ22865 | 225947_at | 0.20 | 0.24 |
| intersex-like (Drosophila) | IXL | NM_017592 | DKFZp434H247 // MED29 | 225708_at | 0.20 | 0.14 |
| nuclear receptor coactivator 5 | NCOA5 | NM_020967 | CIA // bA465L10.6 | 225145_at | 0.20 | 0.17 |
| makorin, ring finger protein, 1 | MKRN1 | NM_013446 | FLJ21334 // RNF61 | 201285_at | 0.20 | 0.22 |
| armadillo repeat containing 8 | ARMC8 | NM_014154 // NM_015396// NM_213654 | HSPC056//MGC10058 // MGC4880 // S863-2 | 203486_s_at | 0.20 | 0.12 |
| RNA binding motif protein 17 | RBM17 | NM_032905 | MGC14439 // SPF45 | 224780_at | 0.21 | 0.13 |
| nucleoporin like 2 | NUPL2 | NM_007342 | CG1 // NLP,-1 // NLP_1 // hCG1 | 204003_s_at | 0.21 | 0.21 |
| myeloid/lymphoid or mixed-lineage leukemia 2 | MLL2 | NM_003482 | AAD10 // ALR | 227527_at | 0.21 | 0.10 |
| lipase, endothelial | LIPG | NM_006033 | EDL // EL // PRO719 | 219181 at | 0.21 | 0.11 |
| coiled-coil domain containing 14 | CCDC14 | NM_022757 | // FLJ41065 // FLJ12892 // FLJ41065 | 225017_at | 0.21 | 0.17 |
| thyroid hormone receptor interactor 4 | TRIP4 | NM_016213 | HsT17391 | 203732_at | 0.21 | 0.23 |
| histone deacetylase 4 | HDAC4 | NM_006037 | HA6116 // KIAA0288 // HDACA // KIAA0288 | 204225_at | 0.21 | 0.21 |
| TAF4 RNA polymerise II, TATA box binding protein (TBP)-associated factor, 135kDa | TAF4 | NM_003185 | FLJ41943//TAF2C // TAF2C1 // TAF4A // TAFII130 // TAFII135 | 213090_s_at | 0.21 | 0.09 |
| zinc finger with KRAB and SCAN domains 1 | ZKSCAN1 | NM_003439 | 9130423L19Rik // KOX18 // MGC138429 // PHZ-37 // ZNF139 // ZNF36 | 214670_at | 0.21 | 0.15 |
| --- | --- | | | 235436_at | 0.21 | 0.19 |
| --- | --- | | | 228106_at | 0.21 | 0.18 |
| Transcribed locus | --- | | | 235609 at | 0.21 | 0.17 |
| peptidylprolyl isomerase domain and WD repeat containing 1 | PPWD1 | NM_015342 | KIAA0073 | 213483_at | 0.21 | 0.14 |
| polycomb group ring finger 1 | PCGF1 | NM_032673 | 2010002K04Rik // FLJ43754 // MGC10882 // NSPC1 // RNF3A-2 // RNF68 | 210023_s_at | 0.21 | 0.18 |
| RALBP1 associated Eps domain containing 1 | REPS1 | NM_031922 | RALBP1 | 224366_s_at | 0.22 | 0.12 |
| chromosome 17 open reading frame 80 | C17orf80 | NM_017941 | FLJ20721 // HLC-8 // MIG3 | 223351 at | 0.22 | 0.21 |
| KIN, antigenic determinant of recA protein homolog (mouse) | KIN | NM_012311 | BTCD // KIN17 | 205664_at | 0.22 | 0.19 |
| PMS1 postmeiotic segregation increased 1 (S. cerevisiae) | PMS1 | NM_000534 | DKFZp781M0253 // HNPCC3 // PMSL1 // hPMS1 | 213677_s_at | 0.22 | 0.17 |
| E2F transcription factor 7 | E2F7 | NM_203394 | - | 228033_at | 0.22 | 0.06 |
| methyltransferase like 4 | METTL4 | NM_022840 | FLJ23017 // HsT661 // MGC117235 | 219698_s_at | 0.22 | 0.18 |
| ninein (GSK3B interacting protein) | NIN | NM_016350 // NM_020921 // NM_182944 // NM_182945//NM_182946 | KIAA1565 | 225921_at | 0.22 | 0.21 |
| angel homolog 2 (Drosophila) | ANGEL2 | NM_144567 | FLJ12793 // KIAA0759L | 221B25_at | 0.22 | 0.17 |
| cyclin E2 | CCNE2 | NM_057735 // NM_057749 | CYCE2 | 205034_at | 0.22 | 0.05 |
| Full-length cDNA clone CSODL007YI24 of B cells (Ramos cell line) Cot 25-normalized of Homo sapiens (human) | --- | | | 226648_at | 0.22 | 0.12 |
| | | | | | | |
| solute carrier family 37 (glycerol-3-phosphate transporter), member 1 | SLC37A1 | NM_018964 | FLJ22340 // G3PP | 218928_s_at | 0.22 | 0.25 |
| tetratricopeptide repeat domain 4 /// chromosome 1 open reading frame 175 | TTC4 | NM_004623 | MGC5097 | 46167_at | 0.22 | 0.09 |
| nucleolar complex associated 3 homolog (S. cerevisiae) | NOC3L | NM_022451 | AD24 // C10orf117 // FAD24 // FLJ12820 | 218889_at | 0.23 | 0.15 |
| microtubule associated serine/threonine kinase-like | MASTL | NM_032844 | FLJ14813 // RP11-85G18.2 // THC2 | 228468_at | 0.23 | 0.11 |
| chromosome 6 open reading frame 136 | C6orf136 | NM_145029 | MGC15854 | 227455_at | 0.23 | 0.15 |
| furry homolog (Drosophila) | FRY | NM_023037 | 13CDNA73 // 214K23.2 // C13orf14 // CG003 // bA207N4.2 // bA37E23.1 | 204072_s_at | 0.23 | 0.10 |
| KIAA0323 | KIAA0323 | NM 015299 | - | 212355_at | 0.23 | 0.19 |
| ribosomal protein S6 kinase, 52kDa, polypeptide 1 | RPS6KC1 | NM_012424 | RPK118 // humS6PKhl | 218909_at | 0.23 | 0.22 |
| zinc finger CCCH-type, antiviral 1-like | ZC3HAV1L | NM_080660 | C7orf39 // MGC14289 | 2282B0_at | 0.23 | 0.04 |
| origin recognition complex, subunit 5-like (yeast) | ORC5L | NM_002553 // NM_181747 | ORC5 // ORC5P // ORC5T | 204957_at | 0.23 | 0.12 |
| PAX interacting (with transcription-activation domain) protein (with transcription-activation domain) protein 1 | PAXIP1 | NM_007349 | CAGF28 // CAGF29 // FLJ41049 // PACIP1 // PAXIP1 L // PTIP // TNRC2 | 212825_at | 0.23 | 0.08 |
| ADP-ribosylhydrolase like 2 | ADPRHL2 | NM_017825 | ARH3 // FLJ20446 // dJ665N4.2 | 223097_at | 0.23 | 0.20 |
| caspase recruitment domain family, member 10 | CARD10 | NM_014550 | BIMP1 // CARMA3 // MGC142219 | 210026_s_at | 0.23 | 0.13 |
| phosphoinositide-3-kinase, class 3 | PIK3C3 | NM 002647 | MGC61518 // Vps34 | 204297_at | 0.23 | 0.22 |
| methyltransferase like 3 | METTL3 | NM_019852 | M6A // MGC4336 // MT-A70 // Spo8 | 209265_s_at | 0.24 | 0.19 |
| chromosome 20 open reading frame 112 | C20orf112 | NM_080616 | DKFZP566G1424 // dJ1184F4.2 | 225224_at | 0.24 | 0.09 |
| transmembrane protein 177 | TMEM177 | NM_030577 | MGC10993 | 218897 at | 0.24 | 0.22 |
| grainyhead-like 1 (Drosophila) | GRHL1 | NM_014552 // NM_198182 | LBP-32 // LBP32 // MGR // TFCP2L2 , | 222830_at | 0.24 | 0.18 |
| FCH domain only 2 | FCH02 | NM_138782 | - | 228220_at | 0.24 | 0.11 |
| Niemann-Pick disease, type C1 | NPC1 | NM 000271 | NPC | 202679_at | 0.24 | 0.20 |
| ATPase, Ca++ transporting, cardiac muscle, slow twitch 2 | ATP2A2 | NM_001681//NM_170665 | ATP2B // DAR // DD // MGC45367 // SERCA2 | 212361_s_at | 0.24 | 0.17 |
| cancer susceptibility candidate 5 | CASC5 | NM_144508 // NM_170589 | AF15Q14 // D40 // | 228323_at | 0.24 | 0.14 |
| Transcribed locus, strongly similar to NP_002194.1 integrin alpha 2 precursor | - | | | 227314_at | 0.24 | 0.12 |
| CDNA FLJ34214 fis, clone FCBBF3021807 | - | | | 227087_at | 0.24 | 0.17 |
| protein phosphatase 1B (formerly 2C), magnesium- dependent, beta isoform | PPM1B | NM_001033556 // NM_001033557 // NM_002706 // NM_177968 // NM_177969 | MGC21657 // PP2C-beta-X PP2CB // PPC2BETAX | 209296_at | 0.24 | 0.07 |
| PHD finger protein 20-like 1 | PHF20L1 | NM-016018 // NM_032205 // NM_198513 | CGI-72 // MGC64923 | 226942_at | 0.24 | 0.10 |
| single-strand-selective monofunctional uracil-DNA glycosylase 1 | SMUG1 | NM_014311 | FDG // HMUDG // MGC104370 // UNG3 | 218685_s_at | 0.24 | 0.24 |
| integrator complex subunit 7 | INTS7 | NM_015434 | C1orf73 // DKFZP4348168 // INT7 | 222250_s_at | 0.24 | 0.20 |
| elongation factor RNA polymerase II-like 3 | ELL3 | NM 025165 | FLJ22637 | 219518_s_at | 0.24 | 0.20 |
| solute carrier family 17 (anion/sugar transporter), member 5 | SLC17A5 | NM_012434 | AST // FLJ22227 // FLJ23268 // ISSD // NSD SD // SIALIN // SIASD // SLD | // 223441_at | 0.24 | 0.18 |
| DnaJ (Hsp40) homolog, subfamily C, member 16 | DNAJC16 | NM_015291 | DKFZp686G1298 // DKFZp686N0387 // DKFZp781I1547 // KIAA0962 | 212908_at | 0.24 | 0.15 |
| CAP-GLY domain containing linker protein 1 | CLIP1 | NM_002956//NM_198240 | CLIP // CLIP-170 // CLIP170 // CYLN1 // MGC131604 // RSN | 201975_at | 0.24 | 0.12 |
| WD repeat domain 26 | WDR26 | NM_025160 | FLJ21016//MIP2 | 218107_at | 0.24 | 0.16 |
| solute carrier family 25 (mitochondrial deoxynucleotide carrier), member 19 | SLC25A19 | NM_021734 | DNC // MCPHA // MUP1 | 223222_at | 0.25 | 0.22 |
| tripartite motif-containing 31 | TRIM31 | NM_007028 // NM_052816 | C6orf13 // HCG1 // HCG1 // RNF | 210159-s-at | 0.25 | 0.21 |
| jub, ajubs homolog (Xenopus laevis) | JUB | NM_032876//NM 198086 | Ajuba//MGC15563 | 225806_at | 0.25 | 0.09 |
| excision repair cross-complementing rodent repair deficiency, complementation group 3 | ERCC3 | NM_000122 | BTF2 // GT.F2H // TFIIH // XPB | RAD25 // 202176_at | 0.25 | 0.22 |
| phosphatase and actin regulator 4 | PHACTR4 | NM_001048183 // NM_001048183 // NM_023923 | DKFZp686L07205 // FLJ13171 // MGC20618 // MGC34186//RP11-442N24 A.1 | 226823_at | 0.25 | 0.15 |

### Example 2: Analysis in PBMC (1)

Since it is not easy to obtain a cancer tissue clinically, the measurement of the marker in hemocytes readily obtainable in peripheral blood would be more useful. Specifically blood was taken from a normal individual (volunteer) and peripheral blood mononuclear cells were purified and treated with compound A, followed by measurement of change in expression of the gene (mRNA) with the microarray (Human Genome U133 plus 2.0 array: Affymetrix).

### (1) Isolation of peripheral blood mononuclear cells (PBMC)

Ficoll-Paque PLUS solution (Amersham, 17-1440-02) was slowly added to the blood taken (with heparin added) from a healthy individual to form a layer underneath the blood (15 ml of Ficoll-Paque PLUS solution was added to 25 ml of blood). After the mixture was centrifuged at 1500 rpm for 30 min, the upper part containing platelets was removed and then a layer containing mononuclear cells was transferred to another tube. The cells were suspended in PBS and centrifuged at 1500 rpm for 5 min, followed by removal of the supernatant. After these steps were repeated twice, the cells were again suspended in RPMI1640 (containing 10% FBS). The number of the cells was then counted.

### (2) Preparation of RNA of PBMC

The cells were suspended in the medium to 5x10⁶ cells/ml and plated 1 ml per well of a 24-well plate. Immediately, 111 µl of compound A (ten times more concentrated than the final concentrations) was added to the well (four wells per each concentration tested). The cells were then cultured in an incubator with 5% carbon dioxide gas at 37°C. Two or four hours later, the supernatant was collected and centrifuged at 1500 rpm for five minutes. TRI reagent (SIGMA) (1 ml) was added to each well of the plate from which the supernatant was removed to harvest the cells, which was added to the centrifuged pellet to dissolve. RNA was purified in accordance with the protocol of TRI reagent. RNA was further purified using RNeasy (QIAGEN). Absorbance at 260 nm was measured to quantify an amount of RNA.

### (3) Analysis of microarray data in PBMC

A .cel file for each sample was obtained by using GeneChip Operating Software Ver.1.2 (Affymetrix). The RMA method was applied to ten of the .cel files obtained in the microarray experiment. Log signal intensity at a gene level was obtained by normalization of that at a probe level. By subtracting a control value from log signal intensity for each case, the log signal intensity was converted into a log signal ratio to the control. Genes were narrowed down in the basis of a log signal ratio, and a signal value. Specifically, among genes showing that the P value was 5% or less; the expression level in the untreated (control) is 100 or more by signal value; and the expression level when treated with 10 nM of compound A both for two and four hours decreased by 50% or more, genes of which expression level decreased to 25% or less upon the two-hour treatment were picked out. The software used is R2.2.1 (http://www.r-project.org/), affy package 1.8.1 (http://www.bioconductor.org).

Among genes showing, upon the treatment with compound A, that the P value was 5% or less; the expression level in the untreated (control) is 100 or more by signal value; and the expression level when treated with 10 nM of compound A both for two and four hours decreased by 50% or more, genes of which expression level decreased to 25% or less upon the two-hour treatment, and results thereof were shown in Table 2. For each gene evaluated, gene name (Gene Name), abbreviated name (Gene Symbol), accession number (Accession), alias name (Synonym), Probe set number in Human Genome U133 Plus 2.0 Array (Human Genome U133 Plus 2.0 Array Probeset ID), the expression level upon the two-hour treatment with E7107 (E7107 (2h)), and the expression level upon the four-hour treatment with E7107 (E7107 (4h)) were respectively shown in Table 2.

**[Table 2]**

| Gene Name | Gene Symbol | Accession | Synonym | Human Genome U133 Plus 2.0 Array | E7107 (2 h) | | E7107 (4 h) | |
|---|---|---|---|---|---|---|---|---|
| | | | | Probeset ID | 10nM | 100nM | 10nM | 100nM |
| oxidised low density lipoprotein (lectin-like) receptor 1 | OLR1 | NM_002543 | CLEC8A // LOX1 // SCARE1 | 210004_at | 0.05 | 0.01 | 0.02 | 0.00 |
| chromosome 15 open reading frame 48 | C15orf48 | NM_03241 // NM_197955 | FLJ22645//FOAP-11 // MGC32925 // NMES1 | 223484_at | 0.06 | 0.02 | 0.01 | 0.01 |
| formyl peptide receptor-like 2 | FPRL2 | NM_002030 | FLM2_HUMAN // FMLPY // FPRH1 // FPRH2 // RMLP-R-I FPRH1 | 230422_at | 0.06 | 0.05 | 0.04 | 0.02 |
| nuclear receptor interacting protein 3 | NRIP3 | NM_020645 | C11orf14 // NY-SAR-105 | 219557_s_at | 0.07 | 0.11 | 0.29 | 0.24 |
| C-type lectin domain family 5, member A | CLEC5A | NM_013252 | CLECSF5// MDL-1// MDL1// MGC138304 | 219890_at | 0.08 | 0.08 | 0.05 | 0.01 |
| colony stimulating factor 2 receptor, alpha, low-affinity (granulocyte-macrophage) | CSF2RA | XM_001133962 // NM_172248 //NM_006140 // NM_172245 NM_172246 // NM_172247 // NM_172249 - | CD116 // CDw116 // CSF2R // CSF2RAX // CSF2RAY // CSF2RX // CSF2RY // GM-CSF-R-alpha // GMCSFR // GMR // MGC3848 // MGC4838 | 210340_s_at | 0.10 | 0.08 | 0.06 | 0.10 |
| tensin 1 | TNS1 | NM_022648 | MGC88584 // TNS | 221748_s_at | 0.10 | 0.09 | 0.09 | 0.06 |
| zinc finger, CCHC domain containing 6 | ZCCHC6 | NM_024617 | DKFZp666B142 // DKFZp686C11112 // DKFZp686F119 // DKFZp68611269 // PAPD6 | 220933_s_at | 0.17 | 0.14 | 0.08 | 0.06 |
| Solute carrier family 43, member 2 | SLC43A2 | NM_152346 | FLJ23848 // LAT4 // MGC34680 | 228918_at | 0.17 | 0.17 | 0.08 | 0.08 |
| trafficking protein particle complex 4 | TRAPPC 4 | NM_016146 | CGI-104 // HSPC172 // PTD009 // SBDN // TRS23 | 217959_s_at | 0.18 | 0.08 | 0.07 | 0.04 |
| general transcription factor IIH, polypeptide 1, 62kDa | GTF2H1 | NM_005316 | BTF2 // TFUH | 202451_at | 0.18 | 0.07 | 0.07 | 0.02 |
| solute carrier family 25 (mitochondrial deoxynucleotide 9 carrier), member 19 | SLC25A1 | NM_021734 | DNC // MCPHA // MUP1 | 223222_at | 0.18 | 0.17 | 0.20 | 0.18 |
| G protein-coupled receptor 84 | GPR84 | NM_020370 | EX33 // GPCR4 | 223767_at | 0.18 | 0.19 | 0.20 | 0.38 |
| heparanase | HPSE | NM_006665 | HPA // HPR1 // HPSE1 // HSE1 222881_et | | 0.18 | 0.13 | 0.29 | 0.11 |
| tumor necrosis factor, alpha-induced protein 6 | TNFAIP6 | NM_007115 | TSG6 | 206026_s_at | 0.19 | 0.07 | 0.06 | 0.02 |
| Ras association (RalGDS/AF-6) and pleckstrin homology domains 1 | RAPH1 | NM_025252// NM_203365 // NM_213589 | ALS2CR18 // ALS2CR9 // KIAA1681 // LPD // PREL2 // RMO1 // RalGDS/AF-6 | 225188_at | 0.19 | 0.05 | 0.18 | 0.10 |
| chromosome 13 open reading frame 31 | C13orf31 | NM_153218 | DKFZp686D11119 // FLJ38725 | 228937_at | 0.19 | 0.04 | 0.11 | 0.06 |
| WD repeat and SOCS box-containing 1 | WSB1 | NM_015626 // NM_134265 | SW1P1 // WSB-1 | 201294_s_at | 0.20 | 0.29 | 0.17 | 0.25 |
| hypothetical protein LOC51315 | LOC5131 5 | | | 233329_s_at | 0.21 | 0.11 | 0.13 | 0.06 |
| cathepsin L1 | CTSL1 | NM_001912 // NM_145918 | CATL // CTSL//FLJ31037//MEP | 202087_s_at | 0.21 | 0.20 | 0.13 | 0.13 |
| AHA1, activator of heat shock 90kDa protein ATPase homolog 2 (yeast) | AHSA2 | NM_152392 | DKPZp564C236 // Hch1 | 226665_at | 0.21 | 0.20 | 0.15 | 0.23 |
| guanine nucleotide binding protein (G protein), alpha 15 (Gq class) | GNA15 | NM_002068 | GNA16 | 205349_at | 0.22 | 0.11 | 0.15 | 0.15 |
| Transcribed locus | - | | | 230098_at | 0.22 | 0.08 | 0.14 | 0.08 |
| chemokine (C-C motif) receptor-like 2 | CCRL2 // 12 | NM_003965 LOC6423 | CKRX // CRAM-A // CRAM-B // MGC116710 | 211434_s_at | 0.22 | 0.22 | 0.35 | 0.29 |
| retinobtastoma binding protein 6 | RBBP6 | NM_006910 // NM_018703// NM_032626 | DKFZp686PO638 // DKFZp761B2423 // MY038 // RBQ-1 | 212781_at | 0.23 | 0.12 | 0.17 | 0.17 |
| Heterogeneous nuclear ribonucleoprotein A2/B1 | HNRPA2 B1 | NM_002137 // NM_031243 | HNRNPA2 // HNRNPB1 // HNRPA2 // HNRPB1 // RNPA2 // SNRPB1 | 225107_at | 0.23 | 0.07 | 0.20 | 0.10 |
| docking protein 3 | DOK3 | NM_024872 | FLJ22570 // FLJ39939 | 223553_s_at | 0.23 | 0.30 | 0.26 | 0.35 |
| NLR family, pyrin domain containing 12 | NLRP12 | NM_033297 // NM_144687 | CLR19.3 // Monarch1 // NALP12 // PAN6 // PYPAF7 // RNO // RNO2 | 1554952_s_ at | 0.23 | 0.24 | 0.26 | 0.20 |
| membrane-spanning 4-domains, subfamily A, member | MS4A7 | NM_021201 // NM_206938 // NM_206939 // NM_206940 | 4SPAN2 // CD20L4 //CFFM4 // MGC22368 // MS4A8 | 224358_s_at | 0.24 | 0.22 | 0.04 | 0.03 |
| non-SMO element 4 homolog A (S. cerevisiae) | NSMCE4 A | A NM_017615 | C10orf86 // FLJ20003 // NSE4A // RP11-500G22.3 // bA500G22 // bA500G22.3 | 228506_at | 0.24 | 0.19 | 0.18 | 0.19 |
| IQ motif containing G | IQCG | NM_032263 | DKFZp434B227//FLJ11667// FLJ23571 | 235347_at | 0.24 | 0.08 | 0.23 | 0.05 |
| ataxin 1 | ATXN1 | NM_000332 | ATX1 // D6S504E // SCA1 | 242230_at | 0.25 | 0.25 | 0.24 | 0.13 |
| dehydrogenase/reductase (SDR family) member 9 | DHRS9 | NM_005771 // NM_199204 | 3alpha-HSD // RDH15//RDHL // RETSDR8 | 223952_x_at | 0.25 | 0.18 | 0.31 | 0.12 |

### Example 3: Analysis in PBMC (2)

Since it is not easy to obtain a cancer tissue clinically, the measurement of the marker in hemocytes readily obtainable in peripheral blood would be more useful. Specifically peripheral blood was taken from three normal individuals (volunteers) and peripheral blood mononuclear cells were purified and treated with compound A, followed by measurement of change in expression of the gene (mRNA) by qPCR. In cases where a decrease in the expression of mRNA is used as a marker, the genes in Table 3 below, as a representative example, were found to be usable.

RNA samples were first prepared in accordance with the steps described in Example 2 (1) and (2).

Subsequently RNA was prepared to 30 ng/µl and cDNA was synthesized by using TaqMan Reverse Transcription Reagents (Applied Biosystems). The expression level of SLC25A19, TRAPPC4, GTF2H1, Id1, EDN1, ZCCHC6, HNRPA2B1, and HSPA9B was amplified with TaqMan Gene Expression Assays (catalog numbers below) (Applied Biosystems) as a probe by using TaqMan Universal PCR Master Mix (Applied Biosystems).

### [Catalog number]

SLC25A19 (Hs00222265_m1)
TRAPPC4 (Hs00211691_m1)
GTF2H1 (Hs00366525_g1)
ID1 (Hs00357821_g1)
EDN1 (Hs00174961_m1)
ZCCHC6 (Hs00612265_m1)
HNRPA2B1 (Hs00242600_m1)
HSPA9B (Hs00269818_m1)
Reagents were prepared in accordance with the protocol, and measurement was carried out with ABI7900 (Applied Biosystems). Measured values were corrected by using the expression level of 18S rRNA (Hs99999901_s1, Applied Biosystems) as an internal control. The expression level of each gene was calculated with the expression level in the cells untreated with compound A as 1. Table 3 shows genes whose expression level in PBMC is 25% or less and their results. In Table 3, for each gene evaluated, abbreviated name (Gene Symbol), accession number (Accession), alias name (Synonym), gene name (Gene Name), and the expression level upon treatment with varied concentration of E7107 (E7107 (nM)) were respectively shown.

**[Table 3]**

| Gene Symbol | Accession | Synonym | Gene Name | E7107(nM) | | | |
|---|---|---|---|---|---|---|---|
| | | | | 0 | 3 | 10 | 30 |
| SLC25A19 | NM_021734 | DNC // MCPHA // MUP1 | solute carrier family 25 (mitochondrial deoxynucleotlde carrier), member 19 | 1 | 0.11 | 0.02 | 0.01 |
| TRAPPC4 | NM_018148 | CG1-104 // HSPC172// PTD009 //SBDN // TRS23 | trafficking protein particle complex 4 | 1 | 0.12 | 0.02 | 0.01 |
| GTF2H1 | NM NM_005316 | BTF2 // TFUH | general transcription factor IIH, general transcription factor UH, polypeptide 1, 62kDa | 1 | 0.22 | 0.07 | 0.05 |
| ID1 | NM_002165 // NM_181353 | ID | inhibitor of DNA binding 1, dominant negative helix-loop-helix protein | 1 | 0.5 | 0.15 | 0.09 |
| EDN1 | NM_001955 | ET1 | endothelin 1 | 1 | 0.45 | 0.2 | 0.11 |
| ZCCHC6 | NM_024617 | DKFZp666B142 // DKFZp686C11112 // DKFZp686F119 // DKFZp686I1269 // PAPD6 | zinc finger, CCHC domain containing 6 | 1 | 0.49 | 0.19 | 0.13 |
| HNRPA2B1 | NM_002137 // NM_031243 | HNRNPA2 // HNRNPB1 // HNRPA2 // HNRPB1 // RNPA2 // SNRPB1 | heterogeneous nuclear ribonucleoprotein A2/B1 | 1 | 0.48 | 0.29 | 0.24 |
| HSPA9 | NM_004134 | CSA // GRP75 // HSPA9B // MGC4500 // MOT // MOT2 // MTHSP75 // PBP74 // mot-2 | heat shock 70kDa protein 9 (mortalin) | 1 | 0.39 | 0.26 | 0.17 |

### Example 4 Analysis in PBC

Since fractionation of hemocytes is required for employing PBMC, use of PBMC in a clinical test is complicated. If change of mRNA is confirmed in whole blood (PBC), such a test would be clinically more useful. However, since it is difficult to culture PBC, it is not easy to monitor the change of mRNA *in vitro.* If expression of mRNA in PBC, like in PBMC, was confirmed, the change of mRNA can be monitored. Accordingly, in regard to the genes of Table 2 and Table 3 confirmed to function as the marker in PBMC (SLC25A19, TRAPPC4, GTF2H1, ID1, EDN1, ZCCHC6, and HNRPA2B1), it is examined whether the expression of mRNA can be detected for RNA obtained by the same RNA purification method as in the clinical setting (Tempus, PAX gene).

### (1) Preparation of RNA with Tempus Blood RNA Tube (Applied Biosystems)

Human peripheral blood was collected in the tube (3ml/tube) and combined with Stabilizing Reagent. In accordance with the protocol of the Tube, RNA was purified by the RNA Blood-DNA Method in ABI 6100 PrepStation (Applied Biosystems). Absorbance at 260nm was measured to quantify an amount of RNA.

### (2) Preparation of RNA with PAXgene Blood RNA Tube (QIAGEN)

Human peripheral blood was collected in the tube (2.5ml/tube), combined with Stabilizing Reagent, and left to stand overnight at room temperature. In accordance with the protocol of the Tube, RNA was purified with PAXgene Blood RNA Kit (QIAGEN). Absorbance at 260nm was measured to quantify an amount of RNA.

### (3) Quantification of the expression level of mRNA

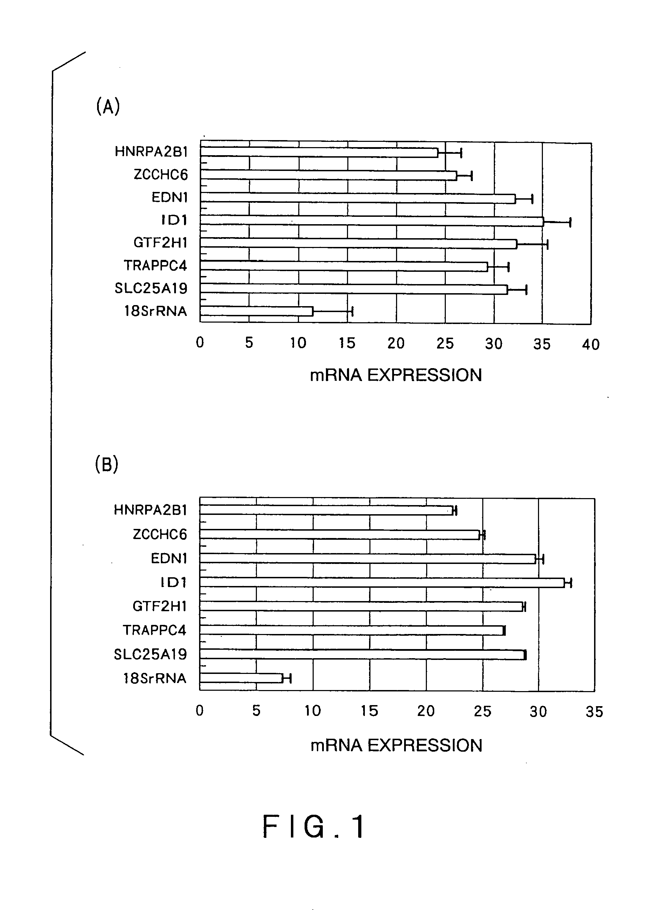

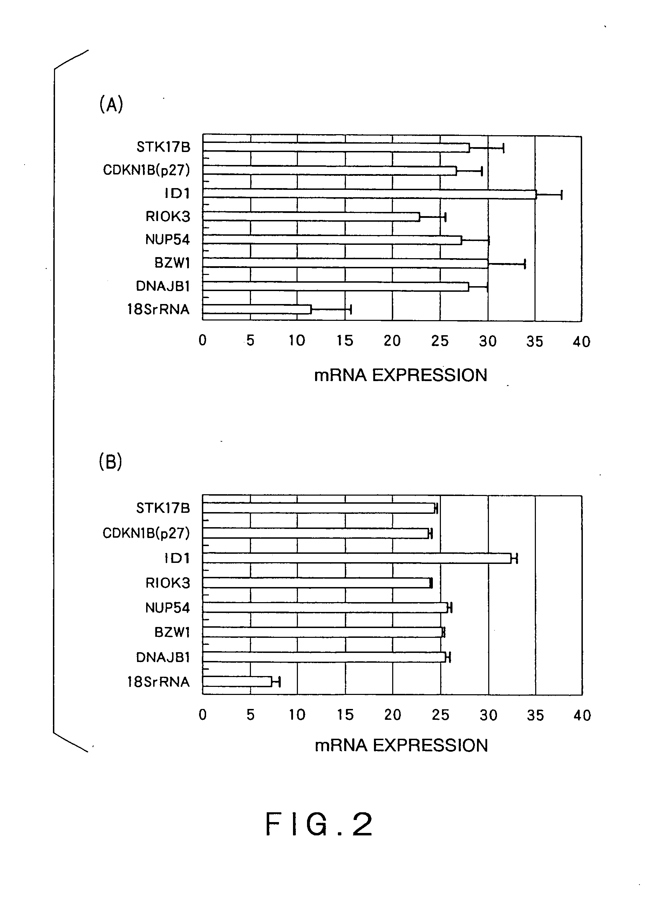

RNA was prepared to 30 ng/µl and cDNA was synthesized by using High Capacity cDNA Reverse Transcription Kits (Applied Biosystems). A probe corresponding respectively to SLC25A19, TRAPPC4, GTF2H1, ID1, EDN1, ZCCHC6, HNRPA2B1 and 18S rRNA was purchased from TaqMan Gene Expression Assays (described above). Reagents were prepared in accordance with the protocol of TaqMan Universal PCR Master Mix (Applied Biosystems) and the expression level of mRNA was measured with ABI7900 (Applied Biosystems). As shown in A (Tempus Blood RNA Tube) and B (PAXgene Blood RNA Tube) in Figure 1 and Figure 2, it was demonstrated that gene expression could sufficiently be confirmed in RNA obtained by both methods.

### Example 5: Analysis in a nude mouse

### (1) Administration of the pladienolide derivative to nude mice subcutaneously transplanted with WiDr human colon carcinoma cells

Nude mice (BALB/cAJcl-nu/nu, 6 weeks old, female) were purchased from CLEA Japan, Inc. After an acclimated period of a week, the mice were subcutaneously transplanted WiDr cells suspended in Hanks' Balanced Salt Solution (GIBCO) (5x10⁶ cells per mouse). Two weeks after the transplantation, when tumor volume was confirmed to grow to more than 200 mm³, the mice were administrated with compound A (30 mg/kg) in a single dose via tail vein injection.

### (2) Blood collection and isolation of the tumor

At the time point of 30 minutes, 1 hour, 2 hours, 4 hours, and 8 hours, after the administration of compound A, two mice per each time point were put down by euthanasia with CO₂. Whole blood (with heparin added) was taken from abdominal aorta of each mouse, TRIzol LS Reagent (Invitrogen) was added thereto, and the mixture was stored at -20°C. After removed, the tumor was stored -20°C in RNAlater (Ambion).

### (3) RNA preparation

RNA preparation from blood was carried out in accordance with the protocol of TRIzol LS Reagent (Invitrogen). The obtained RNA was further purified with RNeasy (QIAGEN). The tumor treated with RNA later (Ambion) was placed in TRI reagent (SIGMA) and grinded by a homogenizer. RNA was purified in accordance with the protocol of TRI reagent. The obtained RNA was further purified using RNeasy (QIAGEN). Absorbance at 260nm was measured to quantify an amount of each RNA.

### (4) Measurement of mRNA expression level in blood

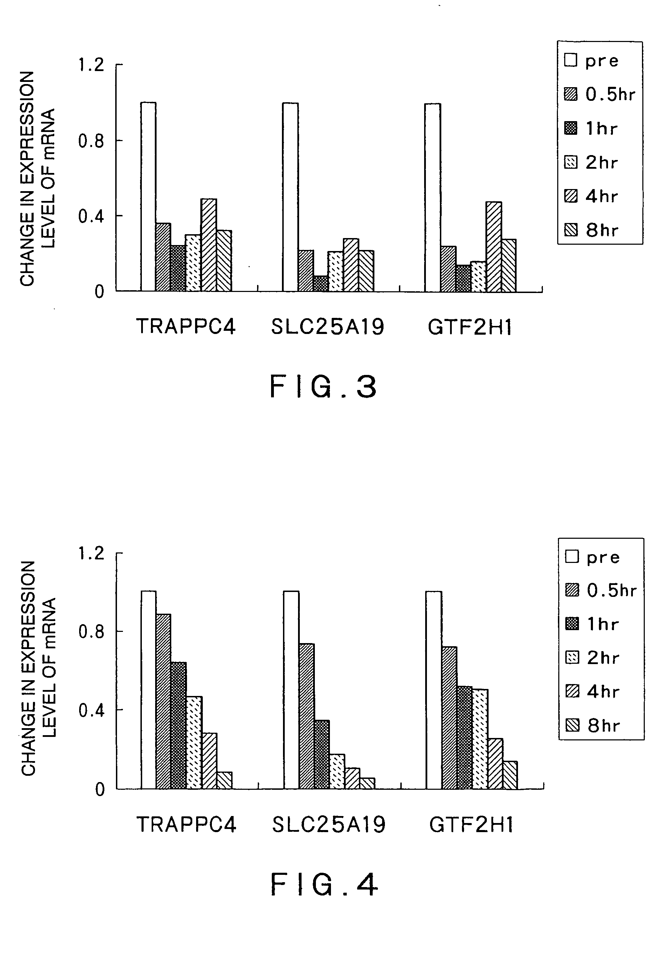

RNA was prepared to 100 ng/µl and cDNA was synthesized using TaqMan Reverse Transcription Reagents (Applied Biosystems). For the expression level of mouse TRAPPC4, mouse SLC25A19, and mouse GTF2H1, the amplification was carried out by using TaqMan Universal PCR Master Mix (Applied Biosystems) with TaqMan Gene Expression Assays (catalog numbers below) (Applied Biosystems) as a probe. Reagents were prepared in accordance with the protocol and the expression level was measured with ABI7900 (Applied Biosystems). Measured values were corrected using the expression level of 18S rRNA (Hs99999901_s1, Applied Biosystems) as an internal control to be calculated the expression level of each gene with the expression level in the group of mice untreated with compound A. Results were shown in Figure 3.

### [Catalog number]

SLC25A19 (Mm01252059_g1)
TRAPPC4 (Mm00445555_m1)
GTF2H1 (Mm00500417_m1)
(5) Measurement of mRNA in tumor
RNA was prepared to 100 ng/µl and cDNA was synthesized using TaqMan Reverse Transcription Reagents (Applied Biosystems). For the expression level of human TRAPPC4, human SLC25A19, human GTF2H1, amplification was carried out by using TaqMan Universal PCR Master Mix(Applied Biosystems) with TaqMan Gene Expression Assays (catalog numbers below) (Applied Biosystems) as a probe. Reagents were prepared in accordance with the protocol and the expression level was measured with ABI7900 (Applied Biosystems). The measurement was corrected using the expression level of 18S rRNA (Hs99999901_s1, Applied Biosystems) as an internal control to be calculated the expression level of each gene with the expression level in the group of mice treated without compound A. Results were shown in Figure 4.

### [Catalog number]

SLC25A19 (Hs00222265_m1)
TRAPPC4 (Hs00211691_m1)
GTF2H1 (Hs00366525_g1)

### Example 6: Analysis in PBMC (2)

Since it is not easy to obtain a cancer tissue clinically, the measurement of the marker in hemocytes readily obtainable in peripheral blood would be more useful. It was then examined whether the marker gene obtained in cancer cells could change in peripheral mononuclear cells in a similar manner as observed in the cancer cells. Specifically peripheral blood was taken from three normal individuals (volunteers) and peripheral mononuclear cells were purified and treated with (8E,12E,14E)-7-((4-Cycloheptylpiperazin-1-yl)carbonyl)oxy-3,6, 16,21-tetrahydroxy-6,10,12,16,20-pentamethyl-18,19-epoxytric osa-8,12,14-trien-11-olide for six hours, followed by measurement of change in expression of the gene by a microarray (Human Exon 1.0 ST Array: Affymetrix). In cases where a change in the expression of the gene is used as a marker, the genes in Table 4 below, as a representative example, were found to be usable.

### (1) Isolation of peripheral blood mononuclear cells (PBMC)

Ficoll-Paque PLUS solution (Amersham, 17-1440-02) was slowly added to the blood taken (with heparin added) from healthy individuals to form a layer underneath the blood (15 ml of Ficoll-Paque PLUS solution was added to 25 ml of blood). After the mixture was centrifuged at 1500 rpm for 30 min, the upper part containing platelets was removed and then a layer containing mononuclear cells was transferred to another tube. The cells were suspended in PBS and centrifuged at 1500 rpm for 5 min, followed by removal of the supernatant. After these steps were repeated twice, the cells were again suspended in RPMI1640 (containing 10% FBS, penicillin, and streptomycin) and the number of the cells was then counted.

### (2) Preparation of total RNA

PBMC was suspended in RPMI1640 medium (containing10% FBS, penicillin, and streptomycin) to 5x10⁶ cells/ml and 1 ml of the suspension was plated per well of a 24-well plate. Immediately, 111 µl of 10 times-concentrated (8E,12E,14E)-7-((4-Cycloheptylpiperazin-1-yl)carbonyl)oxy-3,6, 16,21-tetrahydroxy-6,10,12,16,20-pentamethyl-18,19-epoxytric osa-8,12,14-trien-11-olide was added to the well (six wells per each concentration tested). The cells were then cultured in an incubator with 5% carbon dioxide gas at 37°C. Three hours later, the supernatant was collected and centrifuged at 1500 rpm for five minutes. TRI reagent (SIGMA) (1 ml) was added to each well of the plate from which the supernatant was removed to harvest the cells, which was added to the centrifuged pellet to dissolve. RNA was purified in accordance with the protocol of TRI reagent. RNA was further purified using RNeasy (QIAGEN) and, in accordance with the protocol, DNase I was added to the samples during the procedure. Absorbance at 260 nm was measured to quantify an amount of RNA.

### (3) Analysis of microarray data in PBMC

Ribosomal RNA was removed from 1 µg of each RNA by using RiboMinus Human/Mouse Transcriptome Isolation Kit (Invitrogen). Single stranded cDNA synthesis, double stranded cDNA synthesis, cRNA synthesis, second single stranded cDNA synthesis, cDNA fragmentation, and cDNA labeling in order were carried out for total RNA from which ribosomal RNA was removed, using GeneChip Whole Transcript Sense Target Labeling and Control Reagents (Affymetrix) to make a cDNA probe. Subsequently, the cDNA probe was used for hybridization with Human Exon 1.0 ST Array (Affymetrix). The array was washed and stained, and luminescence intensity was measured by a scanner.

The expression level of the gene was quantified by using Expression Console Ver. 1.0 (Affymetrix) with Summarization Method and Normalization Method being set to "Median polish as used in RMA" and "None", respectively.

Genes showing the change in the expression of the gene in both groups with the 10 nM treatment and the 30 nM treatment is, compared to that in the treated group, less than 25%; the expression level of the gene in the untreated group is more than 100; both P values of t-test for the 10 nM treated group and the untreated group, and for the 30 nM treated group and the untreated group are less than 5% were picked out. (Table 4). In Table 4, for the gene evaluated, gene name (Gene Name), abbreviated name (Gene Symbol), accession number (Accession), alias name (Synonym), transcription cluster number in Human Exon ST 1.0 Array (Trascript Cluster ID), and change in expression level (Fold Change) were respectively shown.

**[Table 4]**

| Gene Name | Gene Symbol | Accession | Synonym | Human Exon ST 1.0 | Fold Change | |
|---|---|---|---|---|---|---|
| | | | | Transcript Cluster ID | 10nM | 30nM |
| reticulocalbin 1, EF-hand calcium binding domain | RCN1 | NM_002901 | FLJ37041//P1G20 // RCAL // RCN | 3325503 | 0.09 | 0.17 |
| oxidized low density lipoprotein (lectin-like) receptor 1 | OLR1 | NM_002543 | CLEC8A // LOX1 // SCARE1 | 3444043 | 0.09 | 0.04 |
| cathepsin L1 | CTSL1 | NM_001912// NM_145918 // | CATL // CTSL // FLJ31037 // MEP | 3178147 | 0.09 | 0.09 |
| tumor necrosis factor (ligand) superfamily, member 15 | TNFSF15 | NM_005118 | MGC129934 // MGC129935 // TL1 // TL1 A // VEGI // VEGI192A | 3222128 | 0.10 | 0.07 |
| kynureninase (L-kynurenine hydrolase) | KYNU | NM_001032998 // NM_003937 | - | 2508520 | 0.10 | 0.10 |
| phospholipase A2, group VII (platelet-activating factor acetylhydrolase, plasma) | PLA2G7 | NM_005084 | LDL-PLA2 // PAFAH | 2955827 | 0.12 | 0.15 |
| solute carrier family 7, (cationic amino acid transporter, y+ system) member 11 | SLC7A11 | NM_014331 | CCBR1 // xCT | 2786322 | 0.13 | 0.06 |
| kynurenine3-monooxygenase (kynurenine3-hydroxylase) | KMO | NM_003679 | dJ317G22.1 | 2388085 | 0.13 | 0.13 |
| nuclear receptor interacting protein 3 | NRIP3 | NM_020645 | C11orf14 // NY-SAR- | 3362159 | 0.15 | 0.13 |
| SLAM family member 7 | SLAMF7 | NM_021181 | CRACC // CS1 CRACC // CS1 | 2363202 | 0.15 | 0.14 |
| dedicator of cytokinesis 4 | DOCK4 | NM_014705 | FLJ34238 // KIAA0716 // MGC134911 // MGC134912 | 3068097 | 0.16 | 0.14 |
| solute carrier family 43, member 3 | SLC43A3 | NM_014096 // NM_017611 // NM_199329 | DKFZp762A227 // EEG1 // FOAP-13 // PRO1659 // SEEEG-1 | 3373845 | 0.17 | 0.14 |
| tumor necrosis factor, alpha-induced protein 6 | TNFAIP6 | NM_007115 | TSG6 | 2510464 | 0.18 | 0.19 |
| CD274 molecule | CD274 | NM_014143 | B7-H // B7H1 // MGC142294 // MGC142296 // PD-L1 // PDCD1L1 // PDCD1LG1 // PDL1 | 3161082 | 0.19 | 0.18 |
| TNF receptor-associated factor 1 | TRAF1 | NM_005658 | EBI6 // MGC:10353 | 3223738 | 0.19 | 0.17 |
| glycerol kinase | GK | NM_000167 // NM_203391 | GK1 // GKD | 3972929 | 0.19 | 0.21 |
| STEAP family member 4 | STEAP4 | NM_024636 | DKFZp666D049 // FLJ23153 // STAMP2 // TIARP // TNFAIP9 | 3060332 | 0.20 | 0.22 |
| leukocyte-associated immunoglobulin-like receptor 1 | LAIR1 | NM_002287 // NM_021706 | CD305// LAIR-1 | 3870824 | 0.21 | 0.14 |
| ADP-ribosylation factor GTPase activating protein 3 | ARFGAP3 | NM_014570 | ARFGAP1 | 3962587 | 0.21 | 0.14 |
| solute carrier family 41, member 2 | SLC41A2 | NM_032148 | DKFZP434K0427 // MGC125331 // MGC125331 // SLC41A1-L1 | 3469180 | 0.21 | 0.17 |
| chromosome 13 open reading frame 31 | C13orf31 | NM_153218 | DKFZP686D11119 // FLJ38725 | 3487600 | 0.21 | 0.19 |
| solute carrier family 37 (glycerol-3-phosphate transporter), member 2 | SLC37A2 | NM_198277 | FLJ00171 // MGC71430 // pp11662 | 3354443 | 0.21 | 0.23 |
| solute carrier family 43, member 2 | SLC43A2 | NM_152346 | FLJ23848 // LAT4 // MGC34680 | 3740367 | 0.22 | 0.18 |
| purinergic receptor P2X, ligand-gated ion channel, 4 | P2RX4 | NM_002560 | P2X4 // P2X4R | 3434760 | 0.22 | 0.22 |
| trafficking protein particle complex 4 | TRAPPC4 | NM_016146 | CGI-104 // HSPC172 // PTD009 // SBDN // TRS23 | 3351775 | 0.23 | 0.21 |
| phosphoinositide-3-kinase, regulatory subunit 5, p101 | PIK3R5 | NM_014308 | F730038115Rik // FOAP-2// P101-PI3K | 3744680 | 0.23 | 0.21 |
| sema domain, transmembrane domain (TM), and cytoplasmic domain, (semaphorin) 6B cytoplasmic domain, | SEMA6B | NM_020241// NM_032108// NM_133327 | SEM-SEMA-Y // SEMA-VIB//SEMAN // Sema VIb //semaZ | 3846860 | 0.23 | 0.22 |
| endoglin (Osler-Rendu-Weber syndrome 1) | ENG | NM_000118 | CD105 // END // FLJ41744 // HHT1 // ORW // ORW1 | 3226097 | 0.24 | 0.20 |

## Claims

1. A method for assaying an action of a compound represented by formula (I), a pharmaceutically acceptable salt thereof, or a solvate of them to a mammal, which comprises detecting a decrease in gene expression level caused by the compound represented by formula (I), the pharmaceutically acceptable salt thereof, or the solvate of them: 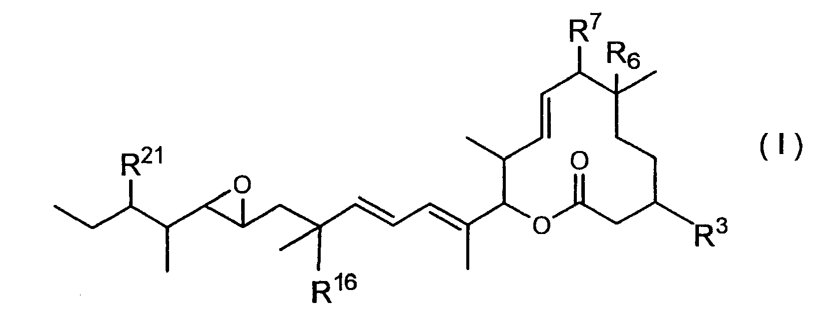 wherein R³, R⁶, R⁷ and R²¹, the same or different, each represents
1) a hydroxyl group or an oxo group formed together with the carbon atom to which it is bound, provided that R⁶ is limited to a hydroxyl group,
2) an optionally substituted C₁₋₂₂ alkoxy group,
3) an optionally substituted unsaturated C₂₋₂₂ alkoxy group,
4) an optionally substituted C₇₋₂₂ aralkyloxy group,
5) an optionally substituted 5- to 14-membered heteroaralkyloxy group,
6) RCO-O- wherein R represents
a) a hydrogen atom,
b) an optionally substituted C₁₋₂₂ alkyl group,
c) an optionally substituted unsaturated C₂₋₂₂ alkyl group,
d) an optionally substituted C₆₋₁₄ aryl group,
e) an optionally substituted 5- to 14-membered heteroaryl group,
f) an optionally substituted C₇₋₂₂ aralkyl group,
g) an optionally substituted 5- to 14-membered heteroaralkyl group,
h) an optionally substituted C₁₋₂₂ alkoxy group,
i) an optionally substituted unsaturated C₂₋₂₂ alkoxy group,
j) an optionally substituted C₆₋₁₄ aryloxy group or
k) an optionally substituted 5- to 14-membered heteroaryloxy group,
7) R^{S1} R^{S2}R^{S3}SiO- wherein R^{S1}, R^{S2} and R^{S3}, the same or different, each represents
a) a C₁₋₆ alkyl group or
b) a C₆₋₁₄ aryl group,
8) a halogen atom,
9) R^{N1}R^{N2}N-R^{M}- wherein R^{M} represents
a) a single bond,
b) -CO-O-,
c) -SO₂-O-,
d) -CS-O-or
e) -CO-NR^{N3}- wherein R^{N3} represents a hydrogen atom or an optionally substituted C₁₋₆ alkyl group, provided that each of the leftmost bond in b) to e)is bound to the nitrogen atom; and R^{N1} and R^{N2}, the same or different from each other and each represents
a) a hydrogen atom,
b) an optionally substituted C₁₋₂₂ alkyl group,
c) an optionally substituted unsaturated C₂₋₂₂ alkyl group,
d) an optionally substituted aliphatic C₂₋₂₂ acyl group,
e) an optionally substituted aromatic C₇₋₁₅ acyl group,
f) an optionally substituted C₆₋₁₄ aryl group,
g) an optionally substituted 5- to 14-membered heteroaryl group,
h) an optionally substituted C₇₋₂₂ aralkyl group,
i) an optionally substituted C₁₋₂₂ alkylsulfonyl group,
j) an optionally substituted C₆₋₁₄ arylsulfonyl group,
k) an optionally substituted 3- to 14-membered non-aromatic heterocyclic group formed by R^{N1} and R^{N2} together with the nitrogen atom to which R^{N1} and R^{N2} are bound, and the non-aromatic heterocyclic group optionally has substituent(s),
I) an optionally substituted 5- to 14-membered heteroaralkyl group,
m) an optionally substituted C₃₋₁₄ cycloalkyl group or
n) an optionally substituted 3- to 14-membered non-aromatic heterocyclic group,
10) R^{N4}SO₂-O- wherein R^{N4} represents
a) an optionally substituted C₁₋₂₂ alkyl group,
b) an optionally substituted C₆₋₁₄ aryl group,
c) an optionally substituted C₁₋₂₂ alkoxy group,
d) an optionally substituted unsaturated C₂₋₂₂ alkoxy group,
e) an optionally substituted C₆₋₁₄ aryloxy group,
f) an optionally substituted 5- to 14-membered heteroaryloxy group,
g) an optionally substituted C₇₋₂₂ aralkyloxy group or
h) an optionally substituted 5- to 14-membered heteroaralkyloxy group,
11) (R^{N5}O)₂PO-O- wherein R^{N5} represents
a) an optionally substituted C₁₋₂₂ alkyl group,
b) an optionally substituted unsaturated C₂₋₂₂ alkyl group,
c) an optionally substituted C₆₋₁₄ aryl group,
d) an optionally substituted 5- to 14-membered heteroaryl group,
e) an optionally substituted C₇₋₂₂ aralkyl group or
f) an optionally substituted 5- to 14-membered heteroaralkyl group),
12) (R^{N1}R^{N2}N)₂PO-O- wherein R^{N1} and R^{N2} have the same meanings as defined above or
13) (R^{N1}RN²N)(R^{N5}O)PO-O- wherein R^{N1}, R^{N2} and R^{N5} have the same meanings as defined above, provided that a compound in which R³, R⁶ ,R⁷ and R²¹ are all hydroxyl groups, and a compound in which R³, R⁶ and R²¹ are all hydroxyl groups and R⁷ is an acetoxy group are excluded,
R¹⁶ represents a hydrogen atom or hydroxyl group.

2. The method according to claim 1, wherein the compound represented by formula (I) is selected from the group consisting of:
(8E,12E,14E)-7-(N-(2-(N',N'-Dimethylamino)ethyl)-N-methylcar bamoyloxy)-3,6,16,21-tetrahydroxy-6,10,12,16,20-pentamethyl -18,19-epoxytricosa-8,12,14-trien-11-olide;
(8E,12E,14E)-3,6,16,21-Tetrahydroxy-6,10,12,16,20-pentameth yl-7-((4-methylhomopiperazin-1-yl)carbonyl)oxy-18,19-epoxytri cosa-8,12,14-trien-11-olide;
(8E,12E,14E)-3,6,16,21-Tetrahydroxy-6,10,12,16,20-pentameth yl-7-((4-methylpiperazin-1-yl)carbonyl)oxy-18,19-epoxytricosa-8,12,14-trien-11-olid e;
(8E,12E,14E)-7-((4-Butylpiperazin-1-yl)carbonyl)oxy-3,6,16,21-tetrahydroxy-6,10,12,16,20-pentamethyl-18,19-epoxytricosa-8, 12,14-trien-11-olide;
(8E,12E,14E)-7-((4-Ethylpiperazin-1-yl)carbonyl)oxy-3,6,16,21-tetrahydroxy-6,10,12,16,20-pentamethyl-18,19-epoxythcosa-8, 12,14-trien-11-olide;
(8E,12E,14E)-3,6,16,21-Tetrahydroxy-6,10,12,16,20-pentameth yl-7-((4-propylpiperazin-1-yl)carbonyl)oxy-18,19-epoxytricosa-8,12,14-trien-11-olide;
(8E,12E,14E)-7-((4-Cyclohexylpiperazin-1-yl)c rbonyl)oxy-3,6,1 6,21-tetrahydroxy-6,10,12,16,20-pentamethyl-18,19-epoxytrico sa-8,12,14-trien-11-olide;
(8E,12E,14E)-7-((4-(Cyclopropylmethyl)piperazin-1-yl)carbonyl) oxy-3,6,16,
21-tetrahydroxy-6,10,12,16,20-pentamethyl-18,19-epoxytricosa -8,12,14-trien-11-olide;
(8E,12E,14E)-3,6,16,21-Tetrahydroxy-6,10,12,16,20-pentameth yl-7-((4-propylhomopiperazin-1-yl)carbonyl)oxy-18,19-epoxytric osa-8,12,14-trien-11-olide;
(8E,12E,14E)-7-((4-(Cyclopropylmethyl)homopiperazin-1-yl)car bonyl)oxy-3,6,
16,21-tetrahydroxy-6,10,12,16,20-pentamethyl-18,19-epoxytric osa-8,12,14-trien-11-olide;
(8E,12E,14E)-7-((4-Cyclopentylpiperazin-1-yl)carbonyl)oxy-3,6, 16,21-tetrahydroxy-6,10,12,16,20-pentamethyl-18,19-epoxytric osa-8,12,14-trien-11-olide;
(8E,12E,14E)-3,6,16,21-Tetrahydroxy-7-((4-isopropylpiperazin-1-yl)carbonyl)oxy-6,10,12,16,20-pentamethyl-18,19-epoxytrico sa-8,12,14-trien-11-olide;
(8E,12E,14E)-7-((4-Cycloheptylpiperazin-1-yl)carbonyl)oxy-3,6, 16,21-Tetrahydroxy-6,10,12,16,20-pentamethyl-18,19-epoxytric osa-8,12,14-trien-11-olide;
(8E,12E,14E)-7-(N-(2-(N',N'-Diethylamino)ethyl)-N-methylcarba moyloxy)-3,6,16,21-tetrahydroxy-6,10,12,16,20-pentamethyl-1 8,19-epoxytricosa-8,12,14-trien-11-olide;
(8E,12E,14E)-3,6,16,21-Tetrahydroxy-7-((4-isobutylhomopipera zin-1-yl)carbonyl)oxy-6,10,12,16,20-pentamethyl-18,19-epoxyt ricosa-8,12,14-trien-11-olide;
(8E,12E,14E)-7-((4-Ethylhomopiperazin-1-yl)carbonyl)oxy-3,6,1 6,21-tetrahydroxy-6,10,12,16,20-pentamethyl-18,19-epoxytrico sa-8,12,14-trien-11-olide;
(8E,12E,14E)-7-((4-Butylhomopiperazin-1-yl)carbonyl)oxy-3,6,1 6,21-tetrahydroxy-6,10,12,16,20-pentamethyl-18,19-epoxytrico sa-8,12,14-trien-11-olide;
(8E,12E,14E)-3,16,21-Trihydroxy-6-methoxy-6,10,12,16,20-pen tamethyl-7-((4-methylpiperazin-1-yl)carbonyl)oxy-18,19-epoxyt ricosa-8,12,14-trien-11-olide;
(8E,12E,14E)-3,16,21-Trihydroxy-6-methoxy-6,10,12,16,20-pen tamethyl-7-((4-(piperidin-1-yl)piperidin-1-yl)carbonyl)oxy-18,1 9-epoxytricosa-8,12,14-trien-11-olide;
(8E,12E,14E)-3,6,7,21-Tetrahydroxy-6,10,12,16,20-pentamethyl -18,19-epoxytricosa-8,12,14-trien-11-olide;
(8E,12E,14E)-7-((4-(2,2-Dimethylpropyl)homopiperazin-1-yl)car bonyl)oxy-3,6,16,21-tetrahydroxy-6,10,12,16,20-pentamethyl-1 8,19-epoxytricosa-8,12,14-trien-11-olide; and
(8E,12E,14E)-3,6,16-Trihydroxy-21-methoxy-6,10,12,16,20-pen tamethyl-7-((4-methylpiperazin-1-yl)carbonyl)oxy-18,19-epoxyt ricosa-8,12,14-trien-11-olide.

3. The method according to claim 1, wherein the detection of the decrease in the gene expression level comprises the steps of:
(a) measuring the expression level of mRNA before and after administration of the compound represented by formula (I), the pharmaceutically acceptable salt thereof ,or the solvate of them to a mammal;
(b) comparing, based on the expression level measured in (a), the expression level of the mRNA before and after administration of the compound represented by formula (I), the pharmaceutically acceptable salt thereof, or the solvate of them, to determine that the compound represented by formula (I), the pharmaceutically acceptable salt thereof, or the solvate of them exerts an action to the mammal when the expression level of mRNA after the administration decreases.

4. The method according to claim 3, wherein the mRNA of which expression level is measured is mRNA of at least one gene selected from the genes listed in Table 1, Table 2, Table 3 and Table 4, or a homologous gene thereof.

5. The method according to claim 4, wherein the gene(s) are selected from TRAPPC4, SLC25A19, GTF2H1, ID1, ZCCHC6 and EDN1.

6. The method according to claim 3, wherein in step (a), the expression level of mRNA in samples obtained from a subject before and after administration of the compound represented by formula (I), the pharmaceutically acceptable salt thereof, or the solvate of them, is measured.

7. The method according to claim 6, wherein the samples obtained from the subject are selected from hemocytes in peripheral blood, plasma and serum.

8. A probe or primer for assaying an action of a compound represented by formula (I), a pharmaceutically acceptable salt thereof, or a solvate of them to a mammal, which consists of a polynucleotide capable of hybridizing with a polynucleotide consisting of a nucleotide sequence of at least one gene selected from the genes listed in Table 1, Table 2, Table 3 and Table 4, or a homologous gene thereof, or a complementary sequence thereof.

9. The probe or primer according to claim 8, which is capable of detecting a genomic intron region or a part thereof in a gene listed in Table 1, Table 2, Table 3 or Table 4, or which is capable of detecting a polynucleotide lacking a part of a genomic exon region in a gene listed in Table 1, Table 2, Table 3 or Table 4.

10. A reagent or kit for assaying an action of a compound represented by formula (I), a pharmaceutically acceptable salt thereof, or a solvate of them to a mammal, which comprises the probe or the primer according to claim 8.

11. The method according to claim 1, wherein the detection of the decrease in the gene expression level comprises the steps of:
(f) measuring the expression level of a protein before and after administration of the compound represented by formula (I), the pharmaceutically acceptable salt thereof, or the solvate of them to a mammal;
(g) comparing, based on the expression level measured in (f), the expression level of the protein before and after administration of the compound represented by formula (I), the pharmaceutically acceptable salt thereof or the solvate of them, to determine that the compound represented by formula (I), the pharmaceutically acceptable salt thereof, or the solvate of them exerts an action to the mammal when the expression level of the protein after the administration decreases.

12. The method according to claim 11, wherein the protein of which expression level is measured is a protein consisting of amino acids encoded by a polynucleotide of at least one gene selected from the genes listed in Table 1, Table 2, Table 3 and Table 4 or a homologous gene thereof.

13. The method according to claim 11, wherein the protein of which expression level is measured is a protein consisting of amino acids encoded by a polynucleotide of at least one gene selected from TRAPPC4, SLC25A19, GTF2H1, ID1, ZCCHC6 and EDN1.

14. The method according to claim 11, wherein in step (f), the expression level of the protein in the samples obtained from a subject before and after administration of the compound represented, by formula (I) or the pharmaceutically acceptable salt thereof or the solvate of them, is measured.

15. The method according to claim 14, wherein the samples obtained from the subject are selected from hemocytes in peripheral blood, plasma and serum.

16. An antibody against an protein consisting of amino acids encoded by a polynucleotide of at least one gene selected from the genes listed in Table 1, Table 2, Table 3 and Table 4 or a homologous gene thereof, or a fragment thereof.

17. A reagent or kit for assaying an action of a compound represented by formula (I), a pharmaceutically acceptable salt thereof, or a solvate of them to a mammal, comprising the antibody or the fragment thereof according to claim 16.
